# EUROPEAN PATENT APPLICATION

(11) **EP 4 844 770 A1**
(43) Date of publication of application: **25.11.2026**
(21) Application number: 25896943.5
(22) Date of filing: 09.10.2025
(51) Int. Cl.: G16B 30/10, G16B 40/00, G06N 3/0455, G06F 18/213, G06F 18/25

(54) **STATE PREDICTION METHOD AND APPARATUS, DEVICE, STORAGE MEDIUM AND PROGRAM PRODUCT**

(30) Priority: 29.11.2024 CN 202411735822
(71) Applicant: Tencent Technology (Shenzhen) Company Limited, Shenzhen, Guangdong 518057 (CN)
(72) Inventor: YAO, Jianhua, Shenzhen, Guangdong 518057 (CN); CAI, Yu, Shenzhen, Guangdong 518057 (CN); QIN, Chenchen, Shenzhen, Guangdong 518057 (CN); ZHAO, Yu, Shenzhen, Guangdong 518057 (CN); HE, Bing, Shenzhen, Guangdong 518057 (CN)
(74) Representative: AWA Sweden AB
(86) International application number: PCT/CN2025/126418
(87) International publication number: WO 2026/113647

(57) **Abstract**

A state prediction method, which is executed by a computer device. The method comprises: acquiring gene sequence information of an object, and comparing the gene sequence information with a reference gene sequence to obtain abnormal sequence information in the gene sequence information, the abnormal sequence information being used for representing the abnormal position and the abnormal type of an abnormal gene unit in the gene sequence information (S202);acquiring detection state information of the object (S204); and inputting the abnormal sequence information and the detection state information into a state prediction model, so as to extract abnormal sequence features of the abnormal sequence information and detection state features of the detection state information, and, on the basis of the abnormal sequence features and the detection state features, obtaining prediction state information of the object (S206) .

## Description

### RELATED APPLICATION

The present disclosure claims priority to Chinese Patent Application No. 202411735822.4, entitled "STATE PREDICTION METHOD AND APPARATUS, DEVICE, STORAGE MEDIUM, AND PROGRAM PRODUCT" filed on November 29, 2024.

### FIELD OF THE TECHNOLOGY

The present disclosure relates to the field of artificial intelligence technologies, and in particular, to a state prediction method and apparatus, a device, a storage medium, and a program product.

### BACKGROUND OF THE DISCLOSURE

In the medical field, a generation process of a genetic state is affected by many factors, such as a genetic factor, an environmental factor, and an emotional factor. In this case, analyzing the generation process of the genetic state is crucial to prediction and treatment of the genetic state.

Often, information about a large amount of genetic samples is usually acquired to construct a statistical table that can represent association relationships between macrofeatures and state labels. The macrofeatures may include regional features and emotional features. In this way, in some embodiments, a state prediction result of an object can be determined from the table according to the macrofeatures of the object. However, accuracy of the state prediction result using the above procedure is relatively low.

### SUMMARY

The present disclosure provides a disease state prediction method and apparatus, a device, a storage medium, and a program product.

The present disclosure provides a disease state prediction method. The method is executable by a computer device, and includes:
obtaining an original gene sequence of an object, and comparing the original gene sequence with a reference gene sequence to obtain abnormal sequence information, the abnormal sequence information comprising locations and types of abnormal gene units in the original gene sequence;
obtaining multi-modal state information of the object; and
extracting, by a state prediction model, an abnormal sequence feature from the abnormal sequence information and a multi-modal state feature from the multi-modal state information, and predicting, by the state prediction model, based on the abnormal sequence feature and the multi-modal state feature, disease state information of the object.

The present disclosure further provides a disease state prediction apparatus. The apparatus includes:
a sequence obtaining module, configured to obtain an original gene sequence of an object, and comparing the original gene sequence with a reference gene sequence to obtain abnormal sequence information, the abnormal sequence information comprising locations and types of abnormal gene units in the original gene sequence;
a state obtaining module, configured to obtain multi-modal state information of the object; and
a state analysis module, configured to extract, by a state prediction model, an abnormal sequence feature from the abnormal sequence information and a multi-modal state feature from the multi-modal state information, and predict, by the state prediction model, based on the abnormal sequence feature and the multi-modal state feature, disease state information of the object.

A computer device is provided, including a memory and a processor, the memory having a computer program stored therein, and the processor, when executing the computer program, implementing operations of the foregoing disease state prediction method.

A computer readable storage medium is provided. The computer readable storage medium has a computer program stored therein, and the computer program, when executed by a processor, implements operations of the foregoing disease state prediction method.

A computer program product is provided. The computer program product includes a computer program, and the computer program, when executed by a processor, implements operations of the foregoing disease state prediction method.

Details of one or more embodiments of the present disclosure are provided in the following accompanying drawings and descriptions. Other features, objectives, and advantages of the present disclosure become apparent with reference to the specification, the accompanying drawings, and the claims.

### BRIEF DESCRIPTION OF THE DRAWINGS

To describe the technical solutions in embodiments of the present disclosure more clearly, the following briefly describes the accompanying drawings required for describing the embodiments. Apparently, the accompanying drawings in the following description show merely embodiments of the present disclosure, and a person of ordinary skill in the art may still derive other drawings from the disclosed accompanying drawings without creative efforts.
FIG. 1 is a diagram of an application environment of a disease state prediction method according to an embodiment.
FIG. 2 is a first schematic flowchart of the disease state prediction method according to an embodiment.
FIG. 3 is a schematic diagram of content of abnormal sequence information according to an embodiment.
FIG. 4 is a first schematic architectural diagram of a state prediction model according to an embodiment.
FIG. 5 is a schematic diagram of content of an abnormal feature matrix according to an embodiment.
FIG. 6 is a second schematic architectural diagram of the state prediction model according to an embodiment.
FIG. 7 is a third schematic architectural diagram of the state prediction model according to an embodiment.
FIG. 8 is a second schematic flowchart of the disease state prediction method according to an embodiment.
FIG. 9 is a third schematic flowchart of the disease state prediction method according to an embodiment.
FIG. 10 is a schematic flowchart of a genetic disease type prediction model according to an embodiment.
FIG. 11 is a structural block diagram of a disease state prediction apparatus according to an embodiment.
FIG. 12 is an internal structural diagram of a computer device according to an embodiment.
FIG. 13 is an internal structural diagram of the computer device according to another embodiment.

### DESCRIPTION OF EMBODIMENTS

The following clearly and completely describes the technical solutions in embodiments of the present disclosure with reference to the accompanying drawings in the embodiments of the present disclosure. Apparently, the described embodiments are some of the embodiments of the present disclosure rather than all of the embodiments. All other embodiments obtained by a person of ordinary skill in the art based on the embodiments in the present disclosure without making creative efforts still fall within the present disclosure. The term, involved in the following description, "some embodiments" describes subsets of all possible embodiments, but "some embodiments" may be the same subset or different subsets of all the possible embodiments and may be combined with each other without conflict.

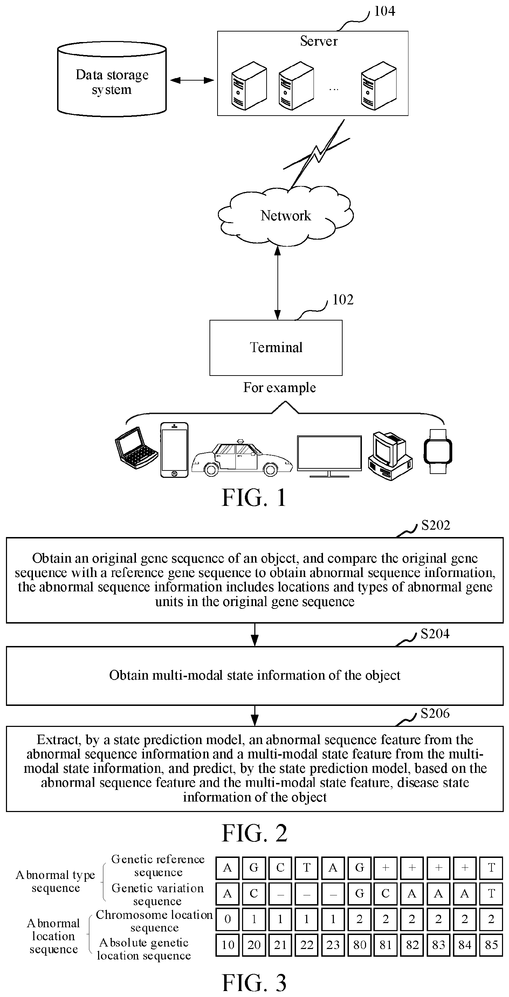

A disease state prediction method provided in embodiments of the present disclosure may be applied to an application environment shown in FIG. 1. A terminal 102 communicates with a server 104 through a network. A data storage system may store data that the server 104 needs to process. The data storage system may be arranged separately, or may be integrated on the server 104, or may be arranged on a cloud or another server. The terminal 102 may be, but is not limited to, various personal computers, notebook computers, smartphones, tablet computers, Internet of Things devices, and portable wearable devices. The Internet of Things device may be a smart speaker, a smart television, a smart air conditioner, a smart in-vehicle device, or the like. The portable wearable device may be a smart watch, a smart band, a head-mounted device, or the like. The head-mounted device may be a virtual reality (VR) device, an augmented reality (AR) device, smart glasses, or the like. The server 104 may be an independent physical server, a server cluster or a distributed system including a plurality of physical servers, or a cloud server providing a cloud computing service.

Both the terminal and the server may be independently configured to perform the disease state prediction method provided in the embodiments of the present disclosure.

For example, the terminal obtains original gene sequence of an object, and compares the original gene sequence with a reference gene sequence to obtain abnormal sequence information in the original gene sequence. The abnormal sequence information is configured to represent locations and types of abnormal gene units in the original gene sequence. The terminal obtains multi-modal state information of the object. The terminal invokes a state prediction model to extract an abnormal sequence feature of the abnormal sequence information and a multi-modal state feature of the multi-modal state information and obtain, based on the abnormal sequence feature and the multi-modal state feature, disease state information of the object.

The terminal and the server may also be cooperatively configured to perform the disease state prediction method provided in the embodiments of the present disclosure.

For example, the terminal obtains original gene sequence of the object, compares the original gene sequence with the reference gene sequence to obtain the abnormal sequence information in the original gene sequence, and transmits the multi-modal state information and the abnormal sequence information of the object to the server. The server invokes the state prediction model to extract the abnormal sequence feature of the abnormal sequence information and the multi-modal state feature of the multi-modal state information and obtain, based on the abnormal sequence feature and the multi-modal state feature, the disease state information of the object. The server returns the disease state information to the terminal.

User information (including, but not limited to, user equipment information, user personal information, and the like) and data (including, but not limited to, data for analysis, stored data, displayed data, and the like) involved in the present disclosure are both information and data that are authorized by a user or that are sufficiently authorized by all parties, and acquisition, use, and processing of related data need to comply with related laws and regulations and standards of related countries and regions.

In an embodiment, as shown in FIG. 2, a disease state prediction method is provided. An example in which the method is applied to a computer device is described for illustration. The computer device may be a terminal or a server. The method may be independently performed by the terminal or the server, or may be implemented through interaction between the terminal and the server. Referring to FIG. 2, the disease state prediction method includes the following operations:

Operation S202: Obtain an original gene sequence of an object, and compare the original gene sequence with a reference gene sequence to obtain abnormal sequence information, the abnormal sequence information includes locations and types of abnormal gene units in the original gene sequence.

In all embodiments of the present disclosure, an object refers to individuals who provide genetic sequences, without presuming disease state. This includes research subjects, individuals, and examinees, such as patients, high-risk populations, healthy individuals, or tested subjects. The original gene sequence is a base sequence representing genetic information of the object, which is specifically a sequence generated by permutation and combination of adenine (A), thymine (T), cytosine (C), and guanine (G). The original gene sequence of the object may be obtained by detecting a sample tissue of the object, for example, a sample such as cells, blood, or hair. In this embodiment of the present disclosure, the original gene sequence may be a specific gene segment of a complete gene sequence of the object, or may be the complete gene sequence of the object.

In some embodiments, the original gene sequence is the complete gene sequence, the original gene sequence of the object may be pre-stored in a data storage system, and the data storage system stores original gene sequence of a plurality of sample objects. In this scenario, in response to a state detection request for an object, the computer device queries a data storage system for and obtains original gene sequence of the object based on an identifier of the object.

In some embodiments, the original gene sequence is a specific gene segment, and the computer device may first obtain the complete gene sequence of the object obtained from the data storage system, and then capture the specific gene segment from the complete gene sequence as the original gene sequence.

The reference gene sequence refers to a standard gene sequence of a species corresponding to an object, and also includes four bases: A, T, C, and G. Although base types of the original gene sequence and the reference gene sequence are the same, in some embodiments, the object is affected by various uncertain factors such as the environment, the emotion, and the living habit, which may cause changes in base positions, a base arrangement mode, and a length of the original gene sequence of the object, that is, genetic variation, and further cause a particular difference between the original gene sequence and the reference gene sequence. That is, the genetic variation refers to a phenomenon in which an object is affected by various uncertain factors such as the environment, the emotion, and the living habit, causing changes in base positions, a base arrangement mode, and a length of the original gene sequence of the object, and further causing a difference between the original gene sequence and the reference gene sequence.

In this embodiment of the present disclosure, an abnormal gene sequence in the original gene sequence is determined by comparing the original gene sequence with the reference gene sequence, and then locations and types of abnormal gene units in the abnormal gene sequence are sorted to obtain abnormal sequence information, so as to represent a genetic feature of the object at a microscopic level with fine granularity. The location of the abnormal gene unit includes a chromosome in which the abnormal gene unit is located and an absolute location of the abnormal gene unit in the reference gene sequence, and the type of the abnormal gene unit includes a current base type of the abnormal gene unit and a normal base type before variation of the abnormal gene unit.

The abnormal gene sequence is determined by comparing the original gene sequence with the reference gene sequence, including abnormal gene units in which genetic variation occurs, and further including normal gene units at loci adjacent to the abnormal gene units in some cases.

In some embodiments, when obtaining the original gene sequence and the reference gene sequence, two gene units of the original gene sequence and the reference gene sequence that are located at a same locus are compared locus by locus, and abnormal gene units that do not match with the reference gene sequence are selected from the original gene sequence, gene units in the reference gene sequence corresponding to the abnormal gene units (i.e., at the same loci) are selected, and locations of the abnormal gene units the original gene sequence are determined. Then, base type identification is performed on the abnormal gene units and the reference gene units to determine types of the abnormal gene units, and the locations of the abnormal gene units are determined. Finally, the types and the locations of the abnormal gene units are included in the abnormal sequence information.

When obtaining the original gene sequence and the reference gene sequence, the two gene units of the original gene sequence and the reference gene sequence that are located at the same locus are compared locus by locus. For two gene units at the same locus, when the gene unit in the original gene sequence is different from the gene unit in the reference gene sequence, it is determined that the gene unit is an abnormal gene unit. When a gene unit exists at a specific locus in the original gene sequence and no gene unit exists at the corresponding locus in the reference gene sequence, the gene unit is an insertion gene unit. When no gene unit exists at a specific locus in the original gene sequence and a gene unit exists at the corresponding locus in the reference gene sequence, the gene unit at this locus in the reference gene sequence is a deletion gene unit. When gene units exist at a specific locus in the original gene sequence and the reference gene sequence, but are of different types, the gene unit at this locus in the original gene sequence is a substitution gene unit. After the abnormal gene unit is determined, the chromosome in which the abnormal gene unit is located and the absolute location of the abnormal gene unit in the reference gene sequence are recorded as the location, and the current base type of the abnormal gene unit and the normal base type before variation are recorded as the type. Finally, the types and the locations of the abnormal gene units are included in the abnormal sequence information.

In some embodiments, when obtaining the original gene sequence and the reference gene sequence, the original gene sequence and the reference gene sequence are compared according to gene segments to determine an abnormal gene segment in the gene sequence, and then, the abnormal gene segment is compared with the reference gene segment locus by locus to determine locations and types of the abnormal gene units in the abnormal gene segment, to obtain the abnormal sequence information.

When obtaining the original gene sequence and the reference gene sequence, the original gene sequence and the reference gene sequence are compared according to gene segments. The original gene sequence and the reference gene sequence are divided into gene segments of equal lengths, and the gene segments at corresponding locations are compared. When the gene segment in the original gene sequence is different from the gene segment in the reference gene sequence, this gene segment is an abnormal gene segment. Next, the abnormal gene segment is compared with the reference gene segment locus by locus, and for each gene unit in the abnormal gene segment, when the gene unit is different from a gene unit at the corresponding locus in the reference gene segment, the gene unit is determined as an abnormal gene unit. When a gene unit exists at a specific locus in the abnormal gene segment and no gene unit exists at the corresponding locus in the reference gene segment, the gene unit is an insertion gene unit. When no gene unit exists at a specific locus in the abnormal gene segment and a gene unit exists at the corresponding locus in the reference gene segment, the gene unit at this locus in the reference gene segment is a deletion gene unit. When gene units exist at a specific locus in the abnormal gene segment and the reference gene segment, but are of different types, the gene unit at this locus in the abnormal gene segment is a substitution gene unit. After the abnormal gene unit is determined, the chromosome in which the abnormal gene unit is located and the absolute location of the abnormal gene unit in the reference gene sequence are recorded as the location, and the current base type of the abnormal gene unit and the normal base type before variation are recorded as the type. Finally, the types and the locations of the abnormal gene units are included in the abnormal sequence information.

In actual application, in addition to the abnormal gene units in which genetic variation occurs, the abnormal gene sequence may further include normal gene units at loci adjacent to the abnormal gene units. In this case, in the abnormal sequence information obtained by sorting the gene units of the abnormal gene sequence, the location of each abnormal gene unit may be represented by using a genetic locus of an adjacent gene unit.

Operation S204: Obtain multi-modal state information of the object.

The multi-modal state information describes a state feature of the object. The state feature includes, but is not limited to, regional information, age, a region of interest image, and the like. Correspondingly, the multi-modal state information is represented in a text form, and/or a value form, and/or an image form.

In some embodiments, in response to a state detection request for an object, the computer device queries a data storage system and obtains multi-modal state information of the object based on an identifier of the object.

In some embodiments, state features of the object in different omics are acquired, and then the state features in different omics are uniformly converted into one format to obtain the multi-modal state information of the object, so as to represent a statistical feature of the object at a macroscopic level with coarse granularity. The state features of the object in different omics may be acquired, for example, regional information, age information, and a region of interest image. For the regional information, a mapping table between regional names and values may be constructed to convert the regional information into corresponding values. For the age information, a value form of the age information is directly reserved. For the region of interest image, an image feature is extracted by using a convolutional neural network (such as ResNet), and an extracted feature vector is used as a value representation of the image. Then, the converted regional value, the age value, and the image feature vector are concatenated to obtain the multi-modal state information of the object, so as to represent a statistical feature of the object at a macroscopic level with coarse granularity.

In an example where the state feature of the object includes the regional information, the age information, and the region of interest image, a value conversion model may be invoked to respectively convert the regional information, the age information, and the region of interest image information into state information in a data format, and then the converted state information is concatenated to obtain the multi-modal state information of the object.

In an example where the state feature of the object includes the regional information, the age information, and the region of interest image, a text conversion model may be invoked to respectively convert the regional information, the age information, and the region of interest image information into state information in a text format, and then the converted state information is concatenated to obtain the multi-modal state information of the object.

Operation S206: Extract, by a state prediction model, an abnormal sequence feature from the abnormal sequence information and a multi-modal state feature from the multi-modal state information, and predict, by the state prediction model, based on the abnormal sequence feature and the multi-modal state feature, disease state information of the object.

The state prediction model is a deep learning model, for example, a transformer model, which supports concurrent computation on different types of input information and integrates features of various types of input information to output the disease state information matching the object. There may be one or more pieces of disease state information, and content of the disease state information includes each predicted state and a probability value of each predicted state.

Specifically, in a state prediction task, the transformer model mainly includes an encoding layer and a decoding layer. The encoding layer includes multi-head self-attention mechanism and a feed-forward network, and is configured to perform feature extraction and encoding on the abnormal sequence information and the multi-modal state information. The multi-head self-attention mechanism may enable the model to pay attention to different parts of the input sequence in different representation subspaces, and a calculation process of the multi-head self-attention mechanism is as follows:

First, a query matrix, a key matrix, and a value matrix are calculated: $Q = {XW}^{Q} , K = {XW}^{K} , V = {XW}^{V}$
where *X* is the input sequence, and *W^{Q}, W^{K},* and *W^{V}* are weight matrixes of query, key, and value respectively.

Then, an attention score is calculated: $Attention \left(Q, K, V\right) = softmax \left(\frac{{QK}^{T}}{\sqrt{{d}_{k}}}\right) V$
where *dₖ* is the dimensionality of the key matrix.

The multi-head self-attention mechanism concatenates outputs of a plurality of attention heads and performs linear transformation: $MultiHead \left(Q, K, V\right) = Concat \left({head}_{1}, ⋯, {head}_{h}\right) {W}^{O}$
where *h* is the number of attention heads, and *W^{O}* is an outputted weight matrix.

The feed-forward network performs nonlinear transformation on an output of the multi-head self-attention mechanism: $FFN \left(x\right) = max \left(0,{xW}_{1}+{b}_{1}\right) {W}_{2} + {b}_{2}$
where *W*₁*, b*₁*, W*₂*,* and *b*₂ are parameters of the feed-forward network.

The decoding layer adds an encoder-decoder attention mechanism based on the encoding layer, and is configured to comprehensively decode an output of the encoding layer and an input of the decoding layer to finally output the disease state information matching the object.

In this embodiment of the present disclosure, the state prediction model represents a complex association relationship between different types of state factors and actual state information of the object. The input of the state prediction model is: the abnormal sequence information and the multi-modal state information of the object. The internal processing logic of the state prediction model is: determining a matching degree between the object and a plurality of pieces of candidate state information with reference to a result of separate analysis on each type of input information and a result of association analysis between different input information. The output of the state prediction model is: one or more pieces of disease state information matching the object and a probability value of each piece of disease state information.

In some embodiments, the state prediction model includes an encoding model and a decoding model. The encoding model is configured to extract features, to obtain an abnormal sequence feature and a multi-modal state feature. The decoding model is configured to decode the extracted features to obtain the disease state information. Specifically, the abnormal sequence feature of the abnormal sequence information and the multi-modal state feature of the multi-modal state information are extracted concurrently by using the encoding model, and the abnormal sequence feature and the multi-modal state feature are analyzed by using the decoding model to obtain the disease state information of the object.

In some embodiments, the state prediction model includes an encoding model and a decoding model. The encoding model is configured for feature extraction and feature fusion to obtain a multi-modal biological feature. The decoding model is configured to decode the fused multi-modal biological feature to obtain the disease state information. Specifically, the abnormal sequence feature of the abnormal sequence information and the multi-modal state feature of the multi-modal state information are extracted concurrently by using the encoding model, the extracted abnormal sequence feature and the multi-modal state feature are fused to obtain a multi-modal biological feature of the object, and the multi-modal biological feature is analyzed by using the decoding model to obtain the disease state information of the object.

Since the abnormal sequence information includes locations and types, the abnormal sequence feature extracted based on the abnormal sequence information also includes a location feature and a type feature. Further, the multi-modal biological feature obtained by fusion based on the abnormal sequence feature and the multi-modal state feature includes the location feature, the type feature, and the multi-modal state feature, so that the state prediction model learns impact of features in various modalities on the state information, and impact of correlation of various features in different modalities on the state information, thereby accurately predicting the state information of the object.

In the foregoing embodiment, the original gene sequence of the object is obtained, and the original gene sequence is compared with the reference gene sequence to obtain the abnormal sequence information in the original gene sequence, the abnormal sequence information being configured for representing locations and types of abnormal gene units in the original gene sequence; the multi-modal state information of the object is obtained; and the abnormal sequence information and the multi-modal state information are input into the state prediction model to extract the abnormal sequence feature of the abnormal sequence information and the multi-modal state feature of the multi-modal state information, and based on the abnormal sequence feature and the multi-modal state feature, the disease state information of the object is obtained. In this way, by performing feature extraction, analysis, and prediction on the original gene sequence and the multi-modal state information of the object by using the preset state prediction model, interference from subjective factors is avoided to some extent, and objectivity of the disease state information is ensured. In addition, the locations and the types of the abnormal gene unit in the original gene sequence are configured for representing of the genetic feature of the object at a microscopic level with fine granularity, and the multi-modal state information is configured to represent the statistical feature of the object at a macroscopic level with coarse granularity. When state prediction is performed, the foregoing information is analyzed by using the state prediction model, which is equivalent to comprehensively considering information about different modalities and different omics of the object, so that the state prediction model extracts more comprehensive multi-modal biological features, thereby ensuring effectiveness of the feature analysis based on the state prediction model, and further improving authenticity and accuracy of the disease state information obtained based on multi-modal biological feature analysis. In addition, the state information prediction operation is performed by the state prediction model deployed on the computer device. To avoid a case in which information identification of the state prediction model fails, before the state prediction model performs the feature extraction and analysis operations, the original gene sequence of the object is processed in advance, so that the abnormal sequence information and the multi-modal state information satisfy the identification requirement of the state prediction model, thereby ensuring normal work of the state prediction model, and further fully using computing resources on the computer device.

In one embodiment, the obtaining an original gene sequence of an object includes: obtaining an initial gene sequence of the object, the initial gene sequence comprising a plurality of gene units at a plurality of loci; and performing quality filtering on the plurality of gene units to obtain the original gene sequence comprising at least one qualified gene unit.

The initial gene sequence may be a base sequence obtained by DNA sequencing, or may be acquired by using a sequencing device. The initial gene sequence includes a plurality of gene units, and each gene unit refers to a single base in the base sequence. A gene unit refers to a single base in a base sequence, and is a basic unit constituting a gene sequence. The initial gene sequence includes a plurality of such gene units.

After the initial gene sequence is obtained, the quality filtering operation is performed to update the initial gene sequence to obtain the original gene sequence. The performing quality filtering on the initial gene sequence refers to an operation of identifying and deleting low-quality genetic data (i.e., gene units) from the initial gene sequence and reserving high-quality gene units.

In some embodiments, the quality filtering includes removing erroneous data. The erroneous data refers to data introduced due to a hardware problem of a test device or a test mode in a process in which the test device tests the initial gene sequence. Specifically, quantitative scoring is performed on the quality of each gene unit in the initial gene sequence according to a preset evaluation mode, and gene units with quantitative scores lower than a preset quality value are deleted from the initial gene sequence, to obtain the original gene sequence.

Specifically, quantitative scoring may be performed on the quality of each gene unit in the initial gene sequence by using a base quality value (Phred quality score) as the preset evaluation mode. A relationship between the base quality value *Q* and a base identification error probability *P* is *Q* = -10log₁₀*P*. Gene units with quantitative scores lower than a preset quality value (for example, *Q* < 20, indicating that the base identification error probability is greater than 1%) are deleted from the initial gene sequence to obtain the original gene sequence.

In some embodiments, the quality filtering includes an operation of removing duplicated data. The duplicated data refers to a plurality of identical or similar gene sequences appearing in the initial gene sequence. Specifically, the initial gene sequence may be divided into a plurality of gene subsequences, a similarity between every two of the gene subsequences is calculated, and any one of two gene subsequences with a similarity greater than a preset similarity threshold is deleted, to obtain the original gene sequence.

Specifically, the initial gene sequence may be divided into a plurality of gene subsequences, and a similarity between every two of the gene subsequences is calculated by using an edit distance (Levenshtein distance). The edit distance refers to a minimum number of times of edit operations (insertions, deletions, or replacements) between two character strings required for conversion from one character string into the other character string. The similarity *S* may be defined as $S = 1 - \frac{d}{max \left(len\left({s}_{1}\right),len\left({s}_{2}\right)\right)}$*,* where *d* is the edit distance, and *len*(*s*₁) and *len*(*s*₂) are respectively lengths of the two gene subsequences.Further, any one of two gene subsequences with a similarity greater than a preset similarity threshold (for example, *S* > 0.9) is deleted, to obtain the original gene sequence.

In the foregoing embodiment, after the initial gene sequence including the gene units at the plurality of loci is obtained, the quality filtering is performed on the initial gene sequence to obtain the original gene sequence. This is equivalent to deleting invalid data of the initial gene sequence to select valid data while considering multiple factors, such as test errors introduced in the process of obtaining the initial gene sequence and redundancy of the initial gene sequence, thereby improving reliability and effectiveness of the original gene sequence and reducing the subsequent computing load of the state prediction model.

In an embodiment, the comparing the original gene sequence with a reference gene sequence to obtain abnormal sequence information in the original gene sequence includes:
aligning the original gene sequence and the reference gene sequence to obtain a candidate gene sequence comprising a plurality of candidate gene units at a part of loci in the original gene sequence;
comparing each of the plurality of candidate gene units with a gene unit at a same location in the reference gene sequence, to determine normal gene units and abnormal gene units in the candidate gene sequence;
constructing an type sequence and an location sequence of the object according to the reference gene sequence, the normal gene units, and the abnormal gene units; and
aligning, according to absolute locations of gene units in the reference gene sequence, the type sequence and the location sequence to obtain the abnormal sequence information.

In some embodiments, the candidate gene sequence of the original gene sequence locates within a candidate locus set (i.e., a part of loci in the original gene sequence). Each gene unit in the candidate gene sequence is compared with a corresponding (i.e., at a same absolute location/locus) gene unit in the reference gene sequence.

In all embodiments, the absolute location is defined in a coordinate system, and a locus is a specific marker point on that coordinate system. A locus corresponds to a specific absolute location. For gene sequence alignment, a locus refers to a specific location on the reference gene sequence (e.g., the 100th base). The sequence alignment refers to aligning two gene sequences according to absolute locations of gene units in the reference gene sequence. The candidate locus set includes a plurality of loci, which may be specifically loci of a plurality of gene units preset based on empirical values. The original gene sequence is queried for gene units at the loci in the candidate locus set, and the plurality of queried gene units are sorted according to the absolute locations of the gene units and then included in the candidate gene sequence.

Each gene unit in the candidate gene sequence is compared with the gene unit at the corresponding locus in the reference gene sequence, and for at any locus in the candidate gene sequence, when the gene unit at this locus in the candidate gene sequence is the same as the gene unit at this locus in the reference gene sequence, it is determined that the gene unit at this locus is a normal gene unit. When the gene unit at this locus in the candidate gene sequence and the gene unit at this locus in the reference gene sequence are of different types, or a gene unit exists at this locus in the candidate gene sequence but no gene unit exists at this locus in the reference gene sequence, it is determined that genetic variation occurs at this locus, and the gene unit at this locus in the candidate gene sequence is an abnormal gene unit. When no gene unit exists at this locus in the candidate gene sequence, it is determined that genetic variation occurs at this locus, and the gene unit at this locus in the reference gene sequence is an abnormal gene unit.

Based on the determination of the normal gene units and the abnormal gene units, first, for each abnormal gene unit, the gene unit before variation and the gene unit after variation thereof are treated as a variant gene combination to obtain a two-dimensional vector corresponding to the abnormal gene unit. For each normal gene unit, the normal gene unit is duplicated to obtain a non-variant gene combination, to obtain a two-dimensional vector corresponding to the normal gene unit.

Next, the absolute location of each abnormal gene unit is determined in the reference gene sequence, and the two-dimensional vector corresponding to each abnormal gene unit is expanded based on the chromosome location and the absolute location of each abnormal gene unit, to obtain a four-dimensional vector corresponding to each abnormal gene unit. The absolute location of each normal gene unit is determined in the reference gene sequence, and the two-dimensional vector corresponding to each normal gene unit is expanded based on the chromosome location and the absolute location of each normal gene unit, to obtain a four-dimensional vector corresponding to each normal gene unit.

Then, while keeping the vector lengths unchanged, the four-dimensional vector corresponding to each abnormal gene unit and the four-dimensional vector corresponding to each normal gene unit are stacked, to construct a fourth-order matrix of the candidate gene sequence. Further, the two vectors representing each gene unit before and after variation are used as the type sequence, and the vectors representing the chromosome location and the absolute location of each gene unit are used as the location sequence.

In an example where the four-dimensional vectors are column vectors, when the candidate gene sequence includes M variant gene sequences and N non-variant gene sequences, the size of the fourth-order matrix of the candidate gene sequence is four rows and (M+N) columns. Each row represents information about one dimension of each gene unit in the candidate gene sequence, and each column represents information about four dimensions of one gene unit, namely, a genetic type before variation, a genetic type after variation, a chromosome location of variation, and an absolute location of variation. In this case, the first row and the second row of the fourth-order matrix are determined as the type sequence, and the third row and the fourth row are determined as location sequence.

In an example where the four-dimensional vectors are row vectors, when the candidate gene sequence includes M variant gene sequences and N non-variant gene sequences, the size of the fourth-order matrix of the candidate gene sequence is four columns and (M+N) rows. Each column represents information about one dimension of each gene unit in the candidate gene sequence, and each row represents information about four dimensions of one gene unit, namely, a genetic type before variation, a genetic type after variation, a chromosome location of variation, and an absolute location of variation. In this case, the first column and the second column of the fourth-order matrix are determined as the type sequence, and the third column and the fourth column are determined as the location sequence.

Finally, the type sequence and the location sequence are sorted according to an ascending order (or a descending order) of the absolute locations of the gene units in the reference gene sequence, to obtain the type sequence and the location sequence that are aligned as the abnormal sequence information.

In the foregoing embodiment, the sequence alignment is performed on the original gene sequence and the reference gene sequence, so as to analyze each gene unit in the original gene sequence. Next, the candidate gene sequence within the candidate locus set is selected from the original gene sequence, so as to further narrow the range for subsequent analysis of gene unit variation and increase the speed of analyzing abnormal genes, thereby quickly and accurately determining the normal gene units and the abnormal gene units in the candidate gene sequence. Then, based on the two dimensions, namely the types and the locations, the variation of each gene unit in the candidate gene sequence is represented with fine granularity to obtain the type sequence and the location sequence, thereby enriching content of the abnormal sequence information.

In an embodiment, the abnormal gene units include at least one of an insertion gene unit, a deletion gene unit, and a substitution gene unit. In an example, the abnormal gene units include insertion gene units, deletion gene units, and substitution gene units. The type sequence includes a genetic reference sequence and a genetic variation sequence. The location sequence includes a chromosome location sequence and an absolute genetic location sequence.

The constructing an type sequence and an location sequence of the object according to the reference gene sequence, the normal gene units, and the abnormal gene units includes:
performing, according to the absolute locations of the gene units in the reference gene sequence, the following operations:
sorting type identifiers of gene units in the reference gene sequence and insertion identifiers of the insertion gene units, to obtain the genetic reference sequence;
sorting type identifiers of the normal gene units, type identifiers of the substitution gene units, type identifiers of the insertion gene units, and deletion identifiers of the deletion gene units, to obtain the genetic variation sequence;
sorting chromosome locations of gene units in the genetic variation sequence and chromosome locations of the deletion gene units, to obtain the chromosome location sequence; and
sorting absolute genetic locations of gene units in the genetic variation sequence and absolute genetic locations of deletion gene units, to obtain the absolute genetic location sequence.

The insertion gene unit refers to a gene unit of the candidate gene sequence at a locus where the gene unit exists in the candidate gene sequence but no gene unit exists in the reference gene sequence at the same locus. The substitution gene unit refers to a gene unit of the candidate gene sequence at a locus where gene units exist in both the candidate gene sequence and the reference gene sequence but are of different types. The deletion gene unit refers to a gene unit of the reference gene sequence at a locus where no gene unit exists in the candidate gene sequence but the gene unit exists in the reference gene sequence at the same locus.

In an example, the abnormal gene units include variant abnormal gene units and normal gene units adjacent to the abnormal gene units, referring to FIG. 3, FIG. 3 is a schematic diagram of abnormal sequence information. The first two rows in FIG. 3 are the abnormal type sequence, and the first row and the second row are respectively a genetic reference sequence and a genetic variation sequence. The last two rows in FIG. 3 are the abnormal location sequence, and the third row and the fourth row are respectively a chromosome location sequence and an absolute genetic location sequence.

In FIG. 3, the genetic reference sequence includes five identifiers, namely, "A", "T", "C", "G", and "+". "A", "T", "C", and "G" represent base types of a gene unit, that is, a type identifier of each gene unit in the reference gene sequence corresponding to the candidate gene sequence, and "+" represents an insertion identifier of an insertion gene unit. The genetic variation sequence includes five identifiers, namely, "A", "T", "C", "G", and "-". "A", "T", "C", and "G" represent base types of a gene unit, and "-" represents a genetic deletion identifier. A value of the chromosome location represents the number of the chromosome where the base is located. A value of the absolute genetic location sequence represents the absolute location of a base in the reference gene sequence.

The insertion identifier is an identifier for representing an insertion gene unit. In the genetic reference sequence, a particular symbol (such as "+") is configured to represent the insertion gene unit. The genetic deletion identifier is an identifier to represent a deletion gene unit. In the genetic variation sequence, a particular symbol (such as "-") is configured to represent genetic deletion.

It can be seen from the abnormal sequence information shown in FIG. 3 that, at a locus whose absolute genetic location is 20, the gene unit of the object variates from G to C, the gene units of the object at loci whose absolute locations are 21 to 23 are deleted, the gene units of the object at loci whose absolute locations are 81 to 84 are newly added, and the insertion gene units are respectively "C", "A", "A", and "A" bases.

In the foregoing embodiment, the insertion gene units of the object are determined by identifying the insertion identifiers in the genetic reference sequence; the deletion gene units of the object are determined by identifying the deletion identifiers in the genetic variation sequence; the base types of the gene units of the object before and after variation are determined by comparing the genetic reference sequence with the genetic variation sequence; and the locations of the insertion gene units, the deletion gene units, and the abnormal gene units in the reference gene sequence can be determined by using the chromosome location sequence and the absolute genetic location sequence.

In an embodiment, the obtaining multi-modal state information of the object includes:
obtaining text information, value information, and image information of the object; mapping, according to a preset conversion relationship, the text information to a text feature of the object; extracting an image feature from the image information; and obtaining, based on the text feature, the image feature, and the value information, the multi-modal state information.

In some embodiments, the image information is vectorized to obtain image feature of the image information, and the value information and the image feature are concatenated to obtain value feature information of the object. Then, the multi-modal state information is obtained based on the text feature and the value feature information.

For example, it is assumed that the preset conversion relationship is a dictionary. For gender information, the dictionary is {'male': 1, 'female': 0}. For whether behavior feature information is present, the dictionary is {'present': 1, 'absent': 0}. Data mapping is performed on the text information according to the dictionary, to obtain the text feature of the object.

During the performing vectorization processing on the image information to obtain image feature of the image information, a pre-trained convolutional neural network (for example, VGG16) may be configured to perform feature extraction on the image information. The image is input into a VGG16 network, and the last fully connected layer is removed to obtain a feature map outputted by an intermediate layer of the network. Then, a global average pooling operation is performed on the feature map to convert the feature map into a one-dimensional vector, to obtain the image feature of the image information.

The text information includes category information of the object, for example, the gender of the object and whether a behavior feature is present. The value information includes living value information of the object, for example, the age and the MSI value of the object. The image information includes a region of interest image of the object, for example, a computed tomography (CT) image or a magnetic resonance imaging (MRI) image.

For the text information, the conversion relationship may be a mapping relationship between text states and values. The text information is converted into a matching value according to the conversion relationship, which is used as the text feature. For example, by using an onehot encoding mode, the gender male is converted into 1, and the gender female is converted into 0. By using the onehot encoding mode, the presence of a behavior feature is converted into 1, and the absence of the behavior feature is converted into 0.

For the value information and the image information, first, the image feature of the image information is extracted, and vectorization processing is performed on the image feature to obtain the feature value of the image information as the image feature. Then, the value information and the image feature are concatenated according to a preset sequence, to obtain the value feature information of the object.

According to a sequence of the text feature prior to the value feature information, the text feature and the value feature information are summarized, that is, the text feature, the value information, and the image feature are concatenated to obtain the multi-modal state information of the object.

The text feature and the value feature information are both in a uniform format and are both represented in a value form, with an objective of facilitating identification and processing by the state prediction model while reserving the multi-dimensional feature information of the object, thereby improving the prediction speed of the state prediction model.

In the foregoing embodiment, data mapping is performed on the text information according to the preset conversion relationship to obtain the text feature of the object. Vectorization processing is performed on the image information to obtain the image feature of the image information, and then the text feature, the value information, and the image feature are included in the multi-modal state information. This is equivalent to unifying forms of the text information and the image information into value representations to comprehensively represent the state information of the object in a plurality of different dimensions in a form of objective data, thereby improving comprehensiveness and accuracy of the multi-modal state information.

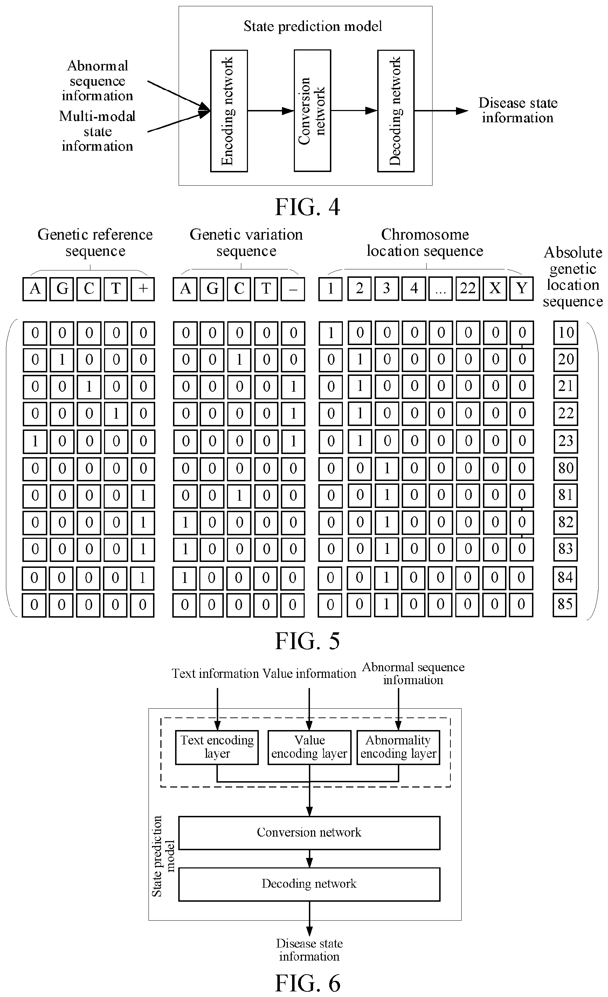

In an embodiment, as shown in FIG. 4, the state prediction model includes an encoding network, a conversion network, and a decoding network. The extracting, by a state prediction model, an abnormal sequence feature from the abnormal sequence information and a multi-modal state feature from the multi-modal state information, and predicting, by the state prediction model, based on the abnormal sequence feature and the multi-modal state feature, disease state information of the object includes:
extracting, based on the encoding network, the abnormal sequence feature of the abnormal sequence information, and extracting the multi-modal state feature of the multi-modal state information; concatenating, based on the conversion network, the abnormal sequence feature and the multi-modal state feature, and performing dimensionality expansion on the concatenated feature to obtain a multi-modal biological feature; and performing, based on the decoding network, dimensionality reduction on the multi-modal biological feature to output the disease state information of the object.

The dimensionality expansion may be implemented by using a multilayer perceptron (MLP). It is assumed that a concatenated feature vector is *x*, the MLP includes two fully connected layers, the first fully connected layer has a weight matrix of *W*₁ and a bias vector of *b*₁, and the second fully connected layer has a weight matrix of *W*₂, and a bias vector of *b*₂.The dimensionality expansion process is: ${h}_{1} = ReLU \left({xW}_{1}+{b}_{1}\right)$ ${h}_{2} = {h}_{1} {W}_{2} + {b}_{2}$
where *ReLU* is an activation function, and *h*₂ is a multi-modal biological feature obtained after dimensionality expansion. The expanded dimension of *h*₂ compared with *x* is determined by the dimension of *W*₂.

In the state prediction model, the state prediction process is as follows: The abnormal sequence information and the multi-modal state information are input into the encoding network to concurrently perform feature extraction operations of the foregoing two types of information, which are specifically extracting the abnormal sequence feature of the abnormal sequence information and extracting the multi-modal state feature of the multi-modal state information. Next, the abnormal sequence feature and the multi-modal state feature that are extracted are input into the conversion network to concatenate the abnormal sequence feature and the multi-modal state feature to obtain a fusion feature, and further, high-dimensional feature extraction is performed on the fusion feature to obtain the multi-modal biological feature of the object. Finally, the multi-modal biological feature is input to the decoding network, dimensionality reduction is performed on the high-dimensional multi-modal biological feature by using the decoding network to obtain probabilities between the object and a preset plurality of pieces of candidate state information, and then the disease state information of the object is determined according to the probabilities between the object and the preset plurality of pieces of candidate state information.

In some embodiments, the plurality of pieces of candidate state information and the probabilities of the candidate state information are summarized and used as the disease state information of the object, or the candidate state information corresponding to the largest probability is used as the disease state information of the object.

In the foregoing embodiment, based on the encoding network, the abnormal sequence feature of the abnormal sequence information is extracted, and the multi-modal state feature of the multi-modal state information is extracted. The abnormal sequence feature and the multi-modal state feature are concatenated based on the conversion network, and dimensionality expansion is performed on the concatenated feature to obtain the multi-modal biological feature; and dimensionality reduction is performed on the multi-modal biological feature based on the decoding network to output the disease state information of the object. In this way, feature extraction is performed by concurrent encoding, which can improve feature extraction efficiency of a model. Next, considering associations between features in different modalities, the extracted features are concatenated to further improve effectiveness between multi-modal biological features, and dimensionality expansion and dimensionality reduction are performed on the concatenated feature to obtain accurate disease state information.

In an embodiment, the encoding network includes a sequence abnormality encoding layer. The abnormal sequence information includes the genetic reference sequence, the genetic variation sequence, the chromosome location sequence, and the absolute genetic location sequence. The extracting, by a state prediction model, an abnormal sequence feature from the abnormal sequence information includes:
identifying the genetic reference sequence and the genetic variation sequence to determine an type of each gene unit in the abnormal sequence information, and generating, based on the type of each gene unit, a type matrix of the abnormal sequence information;
identifying the chromosome location sequence to determine a chromosome location of each gene unit in the abnormal sequence information, and generating, based on the chromosome location of each gene unit, a chromosome location matrix of the abnormal sequence information; and
concatenating the type matrix, the chromosome location matrix, and the absolute genetic location sequence to obtain the abnormal sequence feature.

The input of the sequence abnormality encoding layer is the genetic reference sequence, the genetic variation sequence, the chromosome location sequence, and the absolute genetic location sequence, and the output is the abnormal feature matrix. The sequence abnormality encoding layer may be a linear layer, and the encoding logic is: encoding a plurality of input sequences, and using a unified representation form to describe the variation of different gene units.

The genetic reference sequence and the genetic variation sequence are identified by using the sequence abnormality encoding layer to determine whether each gene unit in the abnormal sequence information is variant. After the abnormal gene units are determined, the type vector of each abnormal gene unit (also named as abnormal type vector) is determined from the preset type vectors according to the abnormal type of the abnormal gene unit, and the normal type vector of each normal gene unit is determined according to the genetic type of the normal gene unit. The abnormal type vector and the normal type vector are of the same dimensionality. Next, the abnormal type vector of each abnormal gene unit and the normal type vector of each normal gene unit are combined to obtain the type matrix of the abnormal sequence information.

The chromosome location sequence is identified by using the sequence abnormality encoding layer to determine the chromosome location of each gene unit in the abnormal sequence information, onehot encoding is respectively performed on the chromosome location of each gene unit to obtain the location vector of each gene unit, and the location vectors of the gene units are combined to obtain the chromosome location matrix of the abnormal sequence information.

The type matrix and the chromosome location matrix of the abnormal sequence information are concatenated by using the sequence abnormality encoding layer. Next, the absolute genetic location of each gene unit in the absolute genetic location sequence is extracted, and the absolute genetic location of each gene unit is concatenated at an adjacent location of the chromosome location vector of the corresponding gene unit to obtain the abnormal feature matrix of the abnormal sequence information, that is, the abnormal sequence feature.

In this embodiment of the present disclosure, type identification, processing, and conversion are performed on the genetic reference sequence and the genetic variation sequence by using the sequence abnormality encoding layer to obtain the type matrix of the abnormal sequence information. The chromosome location sequence is identified and converted by using the sequence abnormality encoding layer to obtain the chromosome location matrix of the abnormal sequence information. The type matrix, the chromosome location matrix, and the absolute genetic location sequence are concatenated to obtain the abnormal feature matrix, and the abnormal feature matrix is determined as the abnormal sequence feature. In this way, in the abnormal sequence feature, the same format is configured to represent the type and the location of each gene unit to standardize the expression of the variation of each gene unit, thereby facilitating subsequent feature fusion of the variant feature and the multi-modal state feature of the gene unit, improving the fusion speed of the conversion network in the state detection model, and further shortening the state prediction time of the state detection model.

In an embodiment, the type matrix includes type vectors of gene units in the abnormal sequence information, and the generating, based on the type of each gene unit, a type matrix of the abnormal sequence information includes:
constructing an initial type vector of the respective gene unit, the initial type vector comprising an insertion gene identifier, a deletion gene identifier, a plurality of reference gene identifiers of different types, and a plurality of variant gene identifiers of different types; setting, when the respective gene unit is an insertion gene unit, a value of the insertion gene identifier and a value of a variant gene identifier matching the type of the insertion gene unit in the initial type vector as a first preset value, and setting values of other identifiers in the initial type vector each as a second preset value, to generate a type vector of the insertion gene unit; setting, when the respective gene unit is a deletion gene unit, a value of the deletion gene identifier and a value of a reference gene identifier matching the type of the deletion gene unit in the initial type vector as the first preset value, and setting values of other identifiers in the initial type vector each as a second preset value, to generate a type vector of the deletion gene unit; and determining, when the respective gene unit is a substitution gene unit, a historical genetic type of the substitution gene unit, setting a value of a reference gene identifier matching the historical genetic type and a value of a variant gene identifier matching the substitution gene unit as the first preset value, and setting values of other identifiers in the initial type vector each as a second preset value, to generate a type vector of the substitution gene unit.

The length of the initial type vector and the content of the vector value for each gene unit are the same, including one insertion gene identifier, one deletion gene identifier, four types of reference gene identifiers (A, G, C, and T), and four types of variant gene identifiers (A, G, C, and T). In addition, the length of the type vector of each gene unit are the same, and the difference between the type vectors of different gene units lies in that specific values in the vectors are different.

When the gene unit is an insertion gene unit, the addition of the gene unit is represented by setting the value of the insertion gene identifier in the initial type vector, and setting the value of the identifier matching the type of the insertion gene unit in the four types of variant gene identifiers (A, G, C, and T). In this embodiment, the value of the insertion gene identifier and the value of the variant gene identifier matching the type of the insertion gene unit are set to the first preset value, for example, 1, and the values of the other identifiers in the initial type vector are set to the second preset value, for example, 0, so that the initial type vector is encoded with values, thereby obtaining the type vector of the gene unit.

When the gene unit is a deletion gene unit, the deletion of the gene unit is represented by setting the value of the deletion gene identifier and setting the value of the identifier matching the type of the deletion gene unit in the four types of reference gene identifiers (A, G, C, and T) in the initial type vector. In this embodiment, the value of the deletion gene identifier and the value of the reference gene identifier matching the type of the deletion gene unit are set to the first preset value, for example, 1, and the values of the other identifiers in the initial type vector are set to the second preset value, for example, 0, so that the initial type vector is encoded with values, thereby obtaining the type vector of the gene unit.

When the gene unit is a substitution gene unit, the historical genetic type of the gene unit, that is, the type of the gene unit before replacement, is determined, and the modification of the gene unit is represented by setting the value of the identifier matching the historical genetic type in the four types of reference gene identifiers (A, G, C, and T), and setting the value of the identifier matching the type of the substitution gene unit in the four types of variant gene identifiers (A, G, C, and T) in the initial type vector. In this embodiment, the value of the reference gene identifier matching the historical genetic type and the value of the variant gene identifier matching the gene unit are set to the first preset value, for example, 1, and the values of the other identifiers in the initial type vector are set to the second preset value, for example, 0, so that the initial type vector is encoded with values, thereby obtaining the type vector of the gene unit.

When the gene unit is not variant, the value of the insertion gene identifier and the value of the identifier matching the type of the insertion gene unit in the four types of variant gene identifiers (A, G, C, and T) in the initial type vector are both set to 0, to represent that the gene unit is not variant.

In an example where the first preset value of 1 and the second preset value of 0 are set for the abnormal sequence information shown in FIG. 3, FIG. 5 is a schematic diagram of an abnormal feature matrix outputted by an encoder. The abnormal feature matrix in FIG. 5 includes 11 rows, and each row represents one gene unit in FIG. 3. The abnormal feature matrix in FIG. 5 includes 35 columns, where the 1^{st} to the 10^{th} columns represent the variation of the gene units, which are respectively the four types of reference gene identifiers (A, G, C, and T), the insertion gene identifier, the four types of variant gene identifiers (A, G, C, and T), and the deletion gene identifier; the 11^{th} to the 34^{th} columns represent the chromosome locations of the gene units, which are 22 autosomes and two sex chromosomes respectively; and the 35^{th} column represents the absolute locations of the gene units.

The gene units represented in the first row in FIG. 5 are the gene units represented in the first column in FIG. 3, that is, the gene units that are not variant. Then, in the first 10 columns of the first row, the value corresponding to each identifier is 0. The gene units in the sixth row and the eleventh row in FIG. 5 are the gene units represented in the sixth column and the eleventh column in FIG. 3, that is, the gene units that are not variant. The specific data setting mode is the same as that described above, and details are not described herein again.

The gene units represented in the second row in FIG. 5 are the gene units represented in the second column in FIG. 3, that is, the gene units that undergo genetic mutation from the base G to the base C. Then, in the first 10 columns of the second row, the values that correspond to the reference gene identifier (the second column) corresponding to the base G and the variant gene identifier (the eighth column) corresponding to the base C are 1, and the values for the other columns are 0.

The gene units represented in the third row in FIG. 5 are the gene units represented in the third column in FIG. 3, that is, the gene units that undergo genetic deletion. Then, in the first 10 columns of the third row, the values that correspond to the reference gene identifier (the third column) corresponding to the deletion gene unit (the base C) and the deletion gene identifier (the tenth column) are 1, and the values for the other columns are 0. The gene units in the fourth row and the fifth row in FIG. 5 are the gene unit represented in the fourth column and the fifth column in FIG. 3, that is, the gene units that undergo genetic deletion. The specific data setting mode is the same as that described above, and details are not described herein again.

The gene units represented in the seventh row in FIG. 5 are the gene units represented in the seventh column in FIG. 3, that is, the insertion gene units. Then, in the first 10 columns of the seventh row, the values that correspond to the insertion gene identifier (the fifth column) and the reference gene identifier (the eighth column) corresponding to the insertion gene unit (the base C) are 1, and the values for the other columns are 0. The gene units in the eighth row to the tenth row in FIG. 5 are the gene units represented in the eighth column to the tenth column in FIG. 3, that is, the insertion gene units. The specific data setting mode is the same as that described above, and details are not described herein again.

In the foregoing embodiment, for any gene unit, the initial type vector of the gene unit is constructed, and the values of the insertion gene identifier, the deletion gene identifier, the plurality of reference gene identifiers of different types, and the plurality of variant gene identifiers of different types in the initial type vector are set to represent the case where the gene units are not variant, and the case where the gene units undergo genetic variation such as addition, deletion, and replacement, so as to standardize the variation of each gene unit.

In an embodiment, the generating, based on the chromosome location of each gene unit, a chromosome location matrix of the abnormal sequence information includes:
constructing an initial location vector of the respective gene unit, the initial location vector comprising a plurality of candidate chromosome identifiers; and
determining, from the plurality of candidate chromosome identifiers, a target chromosome identifier corresponding to the chromosome location of the respective gene unit, and setting a value of the target chromosome identifier as a first preset value, and setting values of other candidate chromosome identifiers in the initial location vector each as a second preset value, to generate a location vector of the respective gene unit in the chromosome location matrix.

No matter whether a gene unit is variant, there is a corresponding chromosome location, and the number of chromosomes of the object is fixed at 24.

Still in the example where the first preset value of 1 and the second preset value of 0 are set for the abnormal sequence information shown in FIG. 3, referring to the abnormal feature matrix shown in FIG. 5, the 11^{th} to the 34^{th} columns in FIG. 5 represent the chromosome locations of the gene units, which are 22 autosomes and two sex chromosomes respectively.

The gene units represented in the first row in FIG. 5 are the gene units represented in the first column in FIG. 3, that is, the gene units whose chromosome location is 0. Then, in the 11^{th} to the 34^{th} columns of the first row, the candidate chromosome identifier in the 11^{th} column is determined as the target chromosome identifier, the value for the 11^{th} column is set to 1, and the value for the 12^{th} to the 34^{th} columns is set to 0.

The gene units represented in the second row in FIG. 5 are the gene units represented in the second column in FIG. 3, that is, the gene units whose chromosome location is 1. Then, in the 11^{th} to the 34^{th} columns of the first row, the candidate chromosome identifier in the 12^{th} column is determined as the target chromosome identifier, the value for the 12^{th} column is set to 1, and the values for the 11^{th} column and the 13^{th} to the 34^{th} columns are set to 0. The gene units in the third row to the fifth row in FIG. 5 are the gene units represented in the third column to the fifth column in FIG. 3. The specific data setting mode is the same as that described above, and details are not described herein again.

The gene units represented in the sixth row in FIG. 5 are the gene units represented in the sixth column in FIG. 3, that is, the gene units whose chromosome location is 2. Then, in the 11^{th} to the 34^{th} columns of the first row, the candidate chromosome identifier in the 13^{th} column is determined as the target chromosome identifier, the value for the 13^{th} column is set to 1, and the values for the 11^{th} to the 12^{th} columns and the 14^{th} to the 34^{th} columns are set to 0. The gene units in the seventh row to the eleventh row in FIG. 5 are the gene units represented in the seventh column to the eleventh column in FIG. 3. The specific data setting mode is the same as that described above, and details are not described herein again.

In this embodiment of the present disclosure, for any gene unit, the initial location vector of the gene unit is constructed, and the value of the plurality of candidate chromosome identifiers in the initial location vector is set to represent the chromosome location of the gene unit, so as to standardize the chromosome location of each gene unit.

In an embodiment, as shown in FIG. 6, the encoding network includes a text encoding layer and a value encoding layer. The extracting the multi-modal state feature of the multi-modal state information includes:
extracting, based on the text encoding layer, a text feature in the multi-modal state information, and extracting, based on the value encoding layer, a value feature in the multi-modal state information; and determining, according to the text feature and the value feature, the multi-modal state feature.

In FIG. 6, the encoding network includes a text encoding layer, a value encoding layer, and a sequence abnormality encoding layer that are disposed in parallel. The text encoding layer includes a linear layer configured to extract text features. In this embodiment, the input of the text encoding layer is the text feature in the multi-modal state information, and the output is the text feature in the multi-modal state information. The value encoding layer includes a linear layer configured to extract value features. In this embodiment, the input of the value encoding layer is the value information in the multi-modal state information, and the output is the value feature in the multi-modal state information. The input of the sequence abnormality encoding layer is the abnormal sequence information, and the output is the abnormal feature matrix. For the content of feature extraction by using onehot encoding, reference may be made to the foregoing embodiment.

The text encoding layer, the value encoding layer, and the sequence abnormality encoding layer extract features concurrently. In this embodiment, the text feature extracted by the text encoding layer and the value feature extracted by the value encoding layer are included in the multi-modal state feature of the multi-modal state information, and the abnormal feature matrix extracted by the sequence abnormality encoding layer is used as the abnormal sequence feature.

In the foregoing embodiment, the text encoding layer is configured to extract the text feature in the multi-modal state information, and the value encoding layer is configured to extract the value feature in the multi-modal state information. This is equivalent to concurrently extracting different types of features in the multi-modal state information by using different encoding layers, to improve the speed of extracting the features in the multi-modal state information, thereby improving efficiency of state prediction by the state prediction model.

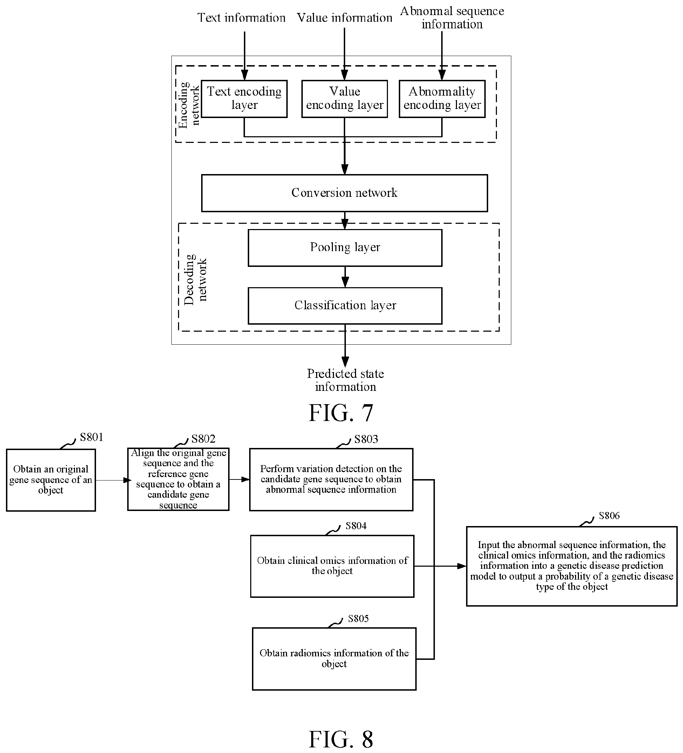

In an embodiment, as shown in FIG. 7, the decoding network includes a pooling layer and a classification layer. The performing, based on the decoding network, dimensionality reduction on the multi-modal biological feature to output the disease state information includes:
performing, based on the pooling layer, dimensionality reduction on the multi-modal biological feature to obtain a one-dimensional abnormal sequence feature of the object; and
predicting, based on the classification layer, a matching probability between the one-dimensional abnormal sequence feature and each piece of candidate state information in a candidate state information set, and determining candidate state information having a matching probability falling within a preset probability interval as the disease state information.

In some embodiments, based on the classification layer, dimensionality reduction on the one-dimensional abnormal sequence feature may be further performed, i.e., the length of the one-dimensional abnormal sequence feature is reduced.

The decoding network in FIG. 7 includes the pooling layer and the classification layer that are connected in series. The pooling layer is connected to the conversion network. The pooling layer may be an average pooling layer, and is configured to perform dimensionality reduction on the multi-modal biological feature outputted by the conversion network to obtain a one-dimensional abnormal sequence feature. The classification layer may include a linear layer, an activation layer, and a linear layer, and is configured to further perform dimensionality reduction on the one-dimensional abnormal sequence feature, predict a matching probability between the object and each piece of candidate state information according to the multi-modal biological feature of the object, and use the candidate state information corresponding to one or more matching probabilities within the preset probability interval as the disease state information.

It is assumed that the multi-modal biological feature is a two-dimensional matrix *M*whose dimensionality is *m* × *n*.The average pooling layer divides the matrix *M* into a plurality of non-overlapping regions, and for each region, an average of elements in the region is calculated as a pooling result of the region.For example, if a *k* × *k* pooling window with a stride of *s* is used, each element *vᵢ* of the pooled one-dimensional abnormal sequence feature vector *v* is calculated as follows: ${v}_{i} = \frac{1}{{k}^{2}} {\sum }_{j = 1}^{k} {\sum }_{l = 1}^{k} {M}_{\left(i\times s+j\right) , \left(i\times s+l\right)}$
where *i* is an index of the pooled vector. In this mode, the multi-modal biological feature is subjected to dimensionality reduction to obtain the one-dimensional abnormal sequence feature.

In an implementable scenario, the candidate state information corresponding to the largest matching probability and the matching probability of the candidate state information may be used as the disease state information of the object, or each piece of candidate state information and the matching probability of each piece of candidate state information are directly summarized as the disease state information.

In the foregoing embodiment, dimensionality reduction is performed on the multi-modal biological feature based on the pooling layer to fully fuse the multi-modal biological feature. Next, dimensionality reduction is performed again on the one-dimensional abnormal sequence feature obtained through the dimensionality reduction based on the classification layer, the matching probability between the object and each piece of candidate state information in the candidate state information set is accurately predicted, and candidate state information having a matching probability falling within the preset probability interval is determined as the disease state information of the object, thereby providing a real and comprehensive determining basis for the state prediction result of the object, and improving accuracy of the state prediction result.

In an embodiment, a method for training the state prediction model includes:
obtaining a state information set, the state information set comprising historical abnormal sequence information, historical multi-modal state information, and historical state information labels of a plurality of sample objects; and
predicting sample disease state information according to the historical abnormal sequence information and the historical multi-modal state information by using the state prediction model, and training, according to loss values between the sample disease state information and the historical state information labels, the state prediction model.

Content of the state information set is constructed based on historical data, and includes actual abnormal sequence information, actual multi-modal state information, and actual state information labels of a plurality of sample objects.

The state prediction model includes an initial encoding network, an initial conversion network, and an initial decoding network. The initial encoding network includes an initial sequence abnormality encoding layer, an initial text encoding layer, and an initial value encoding layer. The initial decoding network includes a pooling layer and an initial classification layer.

The state information set is input into the state prediction model, and the state prediction model learns a logical relationship between the historical abnormal sequence information and the historical multi-modal state information in each sample object, and the historical state information label of the sample object, to obtain the trained state prediction model.

Specifically, the state information set is input to the state prediction model, and the state prediction model is trained by using a stochastic gradient descent algorithm. Assuming that a parameter of the state prediction model is *θ*, for the historical abnormal sequence information *X_{abnormal}* and the historical multi-modal state information *X_{detect}* of each sample object, the model outputs the disease state information *Ŷ* = *f*(*X_{abnormal}, X_{detect}*; *θ*), where *f* is a mapping function of the model. A loss function *L*(*Ŷ*, *Y_{label}*), for example, a cross entropy loss function, is defined to measure a difference between the disease state information *Ŷ* and the historical state information label *Y_{label}.*The gradient ∇*_{θ}L* of the loss function with respect to the parameter *θ* is calculated, and the parameter *θ* = *θ* - *α*∇*_{θ}L* is updated according to the gradient, where *α* is the learning rate. This process is continuously repeated until the value of the loss function converges or reaches a preset quantity of training rounds, to obtain the trained state prediction model.

The training process of the state prediction model means: the state prediction model predicts the sample disease state information according to the historical abnormal sequence information and the historical multi-modal state information, inputs the sample disease state information and the historical state information label to the loss function to obtain a model prediction accuracy, and optimizes the parameter of the state prediction model according to the model prediction accuracy until the loss value is less than a preset threshold, to obtain the trained state prediction model.

In the foregoing embodiment, the state prediction model is trained by using the state information set constructed by using the historical abnormal sequence information, the historical multi-modal state information, and the historical state information labels of the plurality of sample objects, to obtain the trained state prediction model. This end-to-end training mode reduces difficulty in obtaining the state information set, and can provide a more diversified data set for model training, thereby improving the generalization capability of the state prediction model. In addition, the trained state prediction model can be flexibly deployed in various mobile terminals according to actual application requirements, thereby further expanding the range of application scenarios of the state prediction model, and further improving the scenario applicability of the disease state prediction method.

In an embodiment, the state prediction model is a genetic disease prediction model. the abnormal sequence information comprises: a genetic reference sequence, a genetic variation sequence, a chromosome location sequence, and an absolute genetic location sequence of the object; the multi-modal state information comprises: clinical information and image information of the object; and the disease state information comprises: a genetic disease type of the object.

In some embodiments, the disease state prediction method includes:
obtaining the genetic reference sequence, the genetic variation sequence, the chromosome location sequence, and the absolute genetic location sequence of the object; obtaining clinical information and image information of the object; and inputting the genetic reference sequence, the genetic variation sequence, the chromosome location sequence, the absolute genetic location sequence, the clinical information, and the image information into the genetic disease prediction model to extract the multi-modal biological feature of the object, and analyzing the multi-modal biological feature to predict a genetic disease type of the object.

Based on a biological tissue sample of the object, the biological tissue sample is sequenced by using a gene sequencing tool to obtain the initial gene sequence of the object, and then the initial gene sequence is compared with the reference gene sequence to construct the genetic reference sequence, the genetic variation sequence, the chromosome location sequence, and the absolute genetic location sequence of the object.

Original clinical omics information of the object, such as gender, smoking status, and age, is acquired in a questionnaire survey mode, the original clinical omics information, such as gender and smoking status, is digitalized, and the digitalized clinical omics information and the age are used as the clinical information of the object. Original radiomics information of the object, such as an MRI image, is acquired in a medical scanning mode, and feature extraction and vectorization processing are performed on the original radiomics information to obtain image information.

The clinical information, the image information, the genetic reference sequence, the genetic variation sequence, the chromosome location sequence, and the absolute genetic location sequence are sequentially input into the genetic disease prediction model. The genetic disease prediction model extracts the state features in the clinical information and the image information, and extracts the sequence features in the genetic reference sequence, the genetic variation sequence, the chromosome location sequence, and the absolute genetic location sequence. Then, the state features and the sequence features are fused to obtain the multi-modal biological feature of the object. Finally, the multi-modal biological feature is decoded and classified to obtain the probability of the object associated with each genetic disease type, and the genetic disease type corresponding to the largest probability is outputted as the genetic disease type of the object.

For the clinical information, feature extraction is performed by using a linear layer. It is assumed that the clinical information is a vector *x_{clinical}* and the linear layer has a weight matrix of *W_{clinical}* and a bias vector of *b_{clinical},* then the extracted clinical feature is *h_{clinical}* = *W_{clinical}x_{clinical}* + *b_{clinical}.* For the image information, feature extraction is performed by using a convolutional neural network (such as Inception). The image is input into the network to obtain a feature vector *h_{image}* outputted by the network. For the genetic reference sequence, the genetic variation sequence, the chromosome location sequence, and the absolute genetic location sequence, encoding is performed by using a sequence abnormality encoding layer. The specific encoding mode is as described above. The obtained abnormal feature matrix is used as the sequence feature *h_{sequence}*. Then, the clinical feature *h_{clinical},* the image feature *h_{image},* and the sequence feature *h_{sequence}* are concatenated to obtain the multi-modal biological feature *h_{multi-modal}* = [*h_{clinical}*; *h_{image}*; *h_{sequence}*] of the object. Finally, the multi-modal biological feature is decoded and classified by using a decoding network (such as a fully connected layer and an activation function) to obtain the probability of the object associated with each genetic disease type, and the genetic disease type corresponding to the largest probability is outputted as the genetic disease type of the object.

In the foregoing embodiment, the genetic reference sequence, the genetic variation sequence, the chromosome location sequence, the absolute genetic location sequence, the clinical information, and the image information of the object are input into the genetic disease prediction model, so as to instruct the genetic disease prediction model to predict the genetic disease type of the object based on the of the object. In this way, the genetic disease type obtained through prediction by comprehensively considering multiple factors affecting genetic diseases is more accurate.

In an embodiment, the disease state prediction method may be applied to a scenario of genetic disease prediction. As shown in FIG. 8, the method includes the following operations:
Operation S801: Obtain an original gene sequence of an object.

When an initial gene sequence obtained through second-generation or third-generation, gene sequencing is obtained, preprocessing is performed on the initial gene sequence, which includes but is not limited to processing such as quality control and deduplication, to obtain the original gene sequence of the object.

Operation S802: Align the original gene sequence and the reference gene sequence to obtain a candidate gene sequence within a candidate locus set.

Sequence alignment is performed on the original gene sequence and a reference gene sequence. The candidate locus set indicates a plurality of candidate gene units at a part of loci in the original gene sequence
Operation S803: Perform variation detection on the candidate gene sequence to obtain abnormal sequence information.

In some embodiments, the two gene sequences subjected to sequence alignment are tested by using a genetic variation test tool to output a variation test result, and then, based on the variation test result, a genetic reference sequence, a genetic variation sequence, a chromosome location sequence, and an absolute genetic location sequence are constructed as abnormal sequence information.

Operation S804: Obtain clinical omics information of the object.

The clinical omics information includes category features and value features. The category features include gender and smoking status, and the value features include age and microsatellite instability (MSI) score. In actual application, onehot variation processing is configured to digitalize the category features in the clinical omics features, and replace the category features in the clinical omics information with the digitalized category features, to obtain the clinical omics information in a value form.

Operation S805: Obtain radiomics information of the object.

Based on obtaining a region of interest image of the object, feature values of the region of interest image are further obtained as the radiomics information of the object.

Operation S806: Input the abnormal sequence information, the clinical omics information, and the radiomics information into a genetic disease prediction model to output a probability of a genetic disease type of the object.

The genetic disease prediction model may be a transformer model, and specifically, is a multi-modal genetic disease prediction model trained using a historical data set of a large quantity of sample objects before the genetic disease prediction model is actually put into use. The probability of a genetic disease type of the object is the final disease state information of the object.

In the foregoing embodiment, the genetic disease of the object is predicted by using the genetic disease prediction model based on the plurality of types of information such as the clinical information, the genetic variation, and the radiomics of the object, which is equivalent to combining the table features of the object at a macroscopic level and the genetic fine-grained features of the object at a microscopic level, so that the multi-modal genetic disease prediction model learns the degree of impact of various complex factors on the genetic disease, thereby making a more accurate prediction result of the genetic disease.

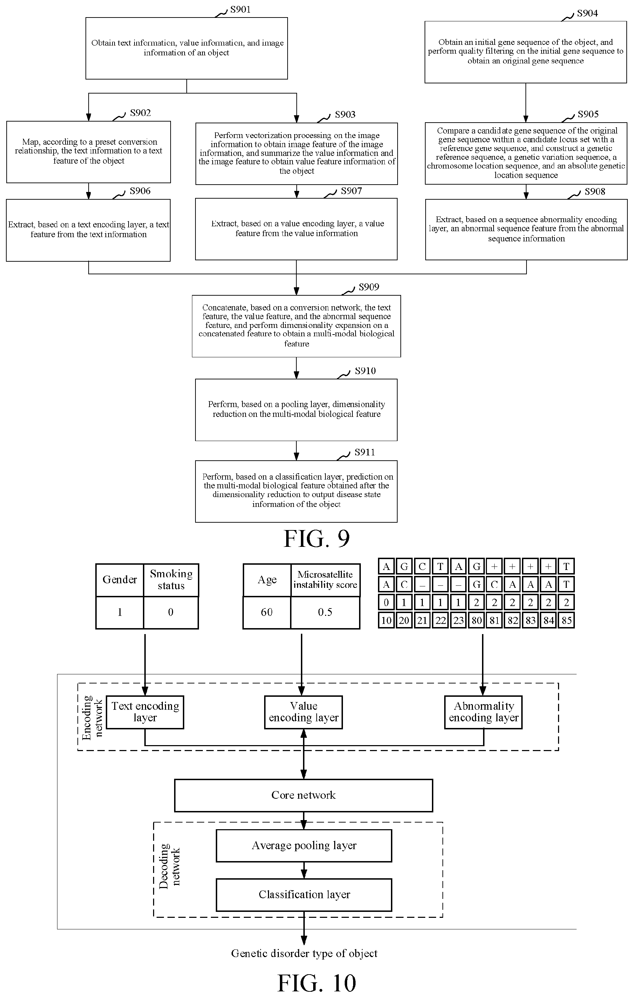

In an embodiment, as shown in FIG. 9, the disease state prediction method includes the following operations:
Operation S901: Obtain text information, value information, and image information of an object.
Operation S902: Map, according to a preset conversion relationship, the text information to a text feature of the object.
Operation S903: Perform vectorization processing on the image information to obtain image feature of the image information, and summarize the value information and the image feature to obtain value feature information of the object.
Operation S904: Obtain an initial gene sequence of the object, and perform quality filtering on the initial gene sequence to obtain an original gene sequence.
Operation S905: Compare a candidate gene sequence of the original gene sequence within a candidate locus set with a reference gene sequence, and construct a genetic reference sequence, a genetic variation sequence, a chromosome location sequence, and an absolute genetic location sequence.
Operation S906: Extract, based on a text encoding layer, a text feature from the text information.
Operation S907: Extract, based on a value encoding layer, a value feature from the value information.
Operation S908: Extract, based on a sequence abnormality encoding layer, an abnormal sequence feature from the abnormal sequence information.
Operation S909: Concatenate, based on a conversion network, the text feature, the value feature, and the abnormal sequence feature, and perform dimensionality expansion on a concatenated feature to obtain a multi-modal biological feature.
Operation S910: Perform, based on a pooling layer, dimensionality reduction on the multi-modal biological feature.
Operation S911: Perform, based on a classification layer, prediction on the multi-modal biological feature obtained after the dimensionality reduction to output disease state information of the object.

Operations S901 to S903 are operations of obtaining the multi-modal state information. Operations S904 to S905 are operations of obtaining the original gene sequence. In some embodiments, processes of obtaining the multi-modal state information and the original gene sequence may be performed in parallel or sequentially. Operations S906 to S911 are a flowchart of state prediction of a preset state prediction model.

In this embodiment of the present disclosure, by performing feature extraction, analysis, and prediction on the original gene sequence and the multi-modal state information of the object by using each network layer in the preset state prediction model, interference from subjective factors can be avoided to some extent, and objectivity of the prediction result of the state prediction model can be ensured. In addition, during the state prediction, the table features of the object at a macroscopic level and the genetic fine-grained features of the object at a microscopic level are comprehensively considered to comprehensively and precisely represent the actual abnormal state of the object, thereby improving authenticity and accuracy of the disease state information obtained by analyzing the multi-modal biological feature.

In a specific embodiment, the disease state prediction method may be applied to a pangenetic disease identification scenario. In this scenario, the state prediction model is a genetic type prediction model for a plurality of types of diseases, and the disease state information is a single genetic disease type.

First, a computer device obtains multi-modal information of an object, namely clinical omics information, radiomics information corresponding to a plurality of positions of the object, and gene sequences corresponding to the plurality of positions of the object, and processes the obtained information to obtain a plurality of types of model input information, so as to facilitate identification and processing by the disease type prediction model.

The clinical omics information includes gender, smoking status, age, and microsatellite instability score. A mode of processing the clinical omics information includes: The gender is digitalized. Specifically, 1 is configured to represent male, and 0 is configured to represent female. The smoking status of the object is digitalized. Specifically, 1 is configured to represent a smoker, and 0 is configured to represent a non-smoker.

The radiomics information includes, but is not limited to, CT scan images of the object's lungs, brain, and the like. The radiomics information is processed. Specifically, an image feature of the CT scan image of the lungs is extracted and vectorized to obtain a first feature vector, and an image feature of the CT scan image of the brain is extracted and vectorized to obtain a second feature vector.

The gene sequences corresponding to the plurality of positions of the object are processed Specifically, candidate gene sequences corresponding to the plurality of positions are extracted from the complete gene sequence of the object by using a variation test tool, and variation testing is performed on the candidate gene sequences to obtain a variation test result; and then, the variation test result is parsed according to a preset conversion condition to construct a genetic reference sequence, a genetic variation sequence, a chromosome location sequence, and an absolute genetic location sequence. The genetic reference sequence, the genetic variation sequence, the chromosome location sequence, and the absolute genetic location sequence are of the same length. In this embodiment of the present disclosure, the foregoing sequences may be summarized into a fourth-order gene sequence matrix.

Then, the digitalized gender, the digitalized smoking status, the age, the microsatellite instability score, the first feature vector, the second feature vector, and the gene sequence matrix are input into the genetic type prediction model to obtain probabilities, outputted by the genetic type prediction model, of the object associated with a plurality of genetic disease types. Finally, the genetic disease type corresponding to the largest probability is used as the genetic disease type of the object.

It is possible to use only one of the clinical omics information and the radiomics information. For example, the clinical omics information and the gene sequences are configured to predict the genetic disease. Still in an example where the gender is male (the mapping value is 1), the smoking status is a non-smoker (the mapping value is 0), the age is 60, and the MSI score is 0.5, referring to FIG. 10, "1", "0", "60", "0.5", and the fourth-order gene sequence matrix are input to the preset genetic disease prediction model. In the schematic architectural diagram of the genetic disease type prediction model shown in FIG. 10, the genetic disease type prediction model performs the following encoding operations concurrently: features corresponding to the gender and the smoking status are extracted based on a text encoding layer, features of the age and the MSI score are extracted based on a value encoding layer, and features of the gene sequence matrix are extracted based on a sequence abnormality encoding layer. Next, the features extracted by the foregoing three encoding layers are fused by using a core network (that is, the conversion network in the foregoing embodiment), and the fused multi-modal biological feature is extracted. The core network includes a plurality of transformer layers. Then, dimensionality reduction is performed on the multi-modal biological feature by using an average pooling layer in a decoding network, matching probabilities between the multi-modal biological feature obtained after the dimensionality reduction and a plurality of genetic disease types are predicted by using a classification layer, and the genetic disease type corresponding to the largest matching probability is outputted as the predicted genetic disease type.

In the foregoing pangenetic disease identification scenario, by comprehensively considering the multi-modal biological feature including the clinical omics information of the object, and the radiomics information and gene sequences corresponding to the plurality of positions of the object, the disease state prediction method provided in this embodiment of the present disclosure precisely positions potential genetic disease information corresponding to the current state of the object, thereby providing a reliable reference basis for subsequent disease prevention.

In a specific embodiment, the disease state prediction method may be applied to a disease identification scenario in a particular biological tissue, such as the lungs. In this scenario, the state prediction model is a pulmonary genetic disease type prediction model, and the disease state information is a pulmonary genetic disease. A computer device obtains the object's gender, smoking status, age, symptom duration, pulmonary image, and candidate gene sequences matching pulmonary diseases. Next, the gender is mapped, where 1 is configured to represent male, and 0 is configured to represent female. The smoking status is mapped, where 1 is configured to represent a smoker, and 0 is configured to represent a non-smoker. The symptom duration is uniformly represented in days. A feature vector of the pulmonary image is extracted. Based on the candidate gene sequences, a pulmonary genetic reference sequence, a pulmonary genetic variation sequence, a pulmonary genetic chromosome location sequence, and a pulmonary absolute genetic location sequence are constructed. Then, the gender data obtained by mapping, the smoking status feature data obtained by mapping, the age, the symptom duration, the feature vector of the pulmonary image, the genetic reference sequence, the pulmonary genetic variation sequence, the pulmonary genetic chromosome location sequence, and the pulmonary absolute genetic location sequence are input into the genetic disease type prediction model. The gender data and the smoking status feature data are encoded by using a category encoding layer of the model to obtain category features. The age, the symptom duration, and the feature vector of the pulmonary image are encoded by using a value encoding layer of the model to obtain value features. The genetic reference sequence, the pulmonary genetic variation sequence, the pulmonary genetic chromosome location sequence, and the pulmonary absolute genetic location sequence are encoded by using an abnormal gene encoding layer of the model to obtain variant features. The category features, the value features, and the variant features are concatenated by using a core network of the model, and a multi-modal biological feature of the object for the lungs is further extracted. Dimensionality reduction is performed on the multi-modal biological feature by using an average pooling layer of the model. Association matching is performed on the feature obtained after the dimensionality reduction and features of a plurality of pulmonary genetic diseases by using a classification module of the model, to obtain a matching degree between the object and each pulmonary genetic disease. The pulmonary genetic disease with the largest matching degree is determined as the predicted pulmonary genetic disease of the object, thereby providing a reliable reference basis for subsequent development of treatment plans for the disease of the object.

Although the operations in the flowcharts involved in the foregoing embodiments are shown sequentially according to arrows, the operations are not necessarily performed sequentially according to the sequence indicated by the arrows. Unless otherwise explicitly stated in this specification, these operations are performed without a strictly limited sequence, and these operations may be performed in another sequence. In addition, at least some operations in the flowcharts in the foregoing embodiments may include a plurality of operations or a plurality of stages. These operations or stages are not necessarily performed at a same moment, but may be performed at different moments. These operations or stages are not necessarily performed sequentially, but may be performed in turn or alternately with other operations or at least some of operations or stages in other operations.

Based on the same inventive concept, an embodiment of the present disclosure further provides a disease state prediction apparatus configured to implement the foregoing related disease state prediction method. An implementation solution provided by the apparatus for resolving the problem is similar to the implementation solution described in the foregoing method. Therefore, for specific definitions of one or more embodiments of the disease state prediction apparatus provided below, reference may be made to the definitions of the disease state prediction method above, and details are not described herein again.

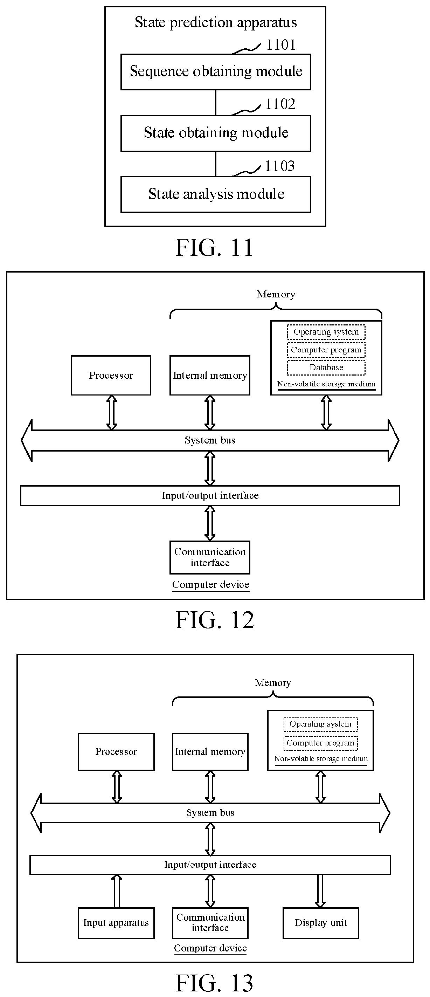

In an embodiment, as shown in FIG. 11, a disease state prediction apparatus is provided, including: a sequence obtaining module 1101, a state obtaining module 1102, and a state analysis module 1103.

The sequence obtaining module 1101 is configured to obtain an original gene sequence of an object, and comparing the original gene sequence with a reference gene sequence to obtain abnormal sequence information, the abnormal sequence information comprising locations and types of abnormal gene units in the original gene sequence.

The state obtaining module 1102 is configured to obtain multi-modal state information of the object.

The state analysis module 1103 is configured to extract, by a state prediction model, an abnormal sequence feature from the abnormal sequence information and a multi-modal state feature from the multi-modal state information, and predict, by the state prediction model, based on the abnormal sequence feature and the multi-modal state feature, disease state information of the object.

In an embodiment, the sequence obtaining module 1101 includes:
an initial sequence obtaining unit, configured to obtain an initial gene sequence of the object, the initial gene sequence including gene units at a plurality of loci; and
a sequence quality filtering unit, configured to perform quality filtering on the plurality of gene units to obtain the original gene sequence comprising at least one qualified gene unit.

In an embodiment, the sequence obtaining module 1101 includes:
a first sequence alignment unit, configured to align the original gene sequence and the reference gene sequence to obtain a candidate gene sequence comprising a plurality of candidate gene units at a part of loci in the original gene sequence;
a gene unit comparing unit, configured to compare each of the plurality of candidate gene units with a gene unit at a same location in the reference gene sequence, to determine normal gene units and abnormal gene units in the candidate gene sequence;
an abnormal sequence construction unit, configured to construct an type sequence and an location sequence of the object according to the reference gene sequence, the normal gene units, and the abnormal gene units; and
a second sequence alignment unit, configured to align, according to absolute locations of gene units in the reference gene sequence, the type sequence and the location sequence to obtain the abnormal sequence information.

In an embodiment, the abnormal gene units comprise insertion gene units, deletion gene units, and substitution gene units; the type sequence comprises a genetic reference sequence and a genetic variation sequence; the location sequence comprises a chromosome location sequence and an absolute genetic location sequence. The abnormal sequence construction unit is configured to perform, according to the absolute locations of the gene units in the reference gene sequence, the following operations:
sorting type identifiers of gene units in the reference gene sequence and insertion identifiers of the insertion gene units, to obtain the genetic reference sequence;
sorting type identifiers of the normal gene units, type identifiers of the substitution gene units, type identifiers of the insertion gene units, and deletion identifiers of the deletion gene units, to obtain the genetic variation sequence;
sorting chromosome locations of gene units in the genetic variation sequence and chromosome locations of the deletion gene units, to obtain the chromosome location sequence; and
sorting absolute genetic locations of gene units in the genetic variation sequence and absolute genetic locations of deletion gene units, to obtain the absolute genetic location sequence

In an embodiment, the state obtaining module 1102 includes:
an information obtaining unit, configured to obtain text information, value information, and image information of the object;
an information mapping unit, configured to map, according to a preset conversion relationship, the text information to a text feature of the object;
a vectorization processing unit, configured to extract an image feature from the image information; and
an information summarization unit, configured to obtain, based on the text feature, the image feature, and the value information, the multi-modal state information.
In an embodiment, the state prediction model includes an encoding network, a conversion network, and a decoding network. The state analysis module 1103 includes:
a feature encoding unit, configured to extract, based on the encoding network, the abnormal sequence feature and the multi-modal state feature;
a feature concatenating unit, configured to concatenate, based on the conversion network, the abnormal sequence feature and the multi-modal state feature, and performing dimensionality expansion on the concatenated feature to obtain a multi-modal biological feature; and
a feature decoding unit, configured to perform, based on the decoding network, dimensionality reduction on the multi-modal biological feature to output the disease state information of the object.

In an embodiment, the feature encoding unit is further configured to identify the genetic reference sequence and the genetic variation sequence to determine an type of each gene unit in the abnormal sequence information, and generate, based on the type of each gene unit, a type matrix of the abnormal sequence information; identify the chromosome location sequence to determine a chromosome location of each gene unit in the abnormal sequence information, and generate, based on the chromosome location of each gene unit, a chromosome location matrix of the abnormal sequence information; and concatenate the type matrix, the chromosome location matrix, and the absolute genetic location sequence to obtain the abnormal sequence feature.

In an embodiment, the feature encoding unit is further configured to construct, an initial type vector of the respective gene unit, the initial type vector comprising an insertion gene identifier, a deletion gene identifier, a plurality of reference gene identifiers of different types, and a plurality of variant gene identifiers of different types; set, when the respective gene unit is an insertion gene unit, a value of the insertion gene identifier and a value of a variant gene identifier matching the type of the insertion gene unit in the initial type vector as a first preset value, and set values of other identifiers in the initial type vector each as a second preset value, to generate a type vector of the insertion gene unit; set, when the respective gene unit is a deletion gene unit, a value of the deletion gene identifier and a value of a reference gene identifier matching the type of the deletion gene unit in the initial type vector as the first preset value, and set values of other identifiers in the initial type vector each as a second preset value, to generate a type vector of the deletion gene unit; and determine, when the respective gene unit is a substitution gene unit, a historical genetic type of the substitution gene unit, set a value of a reference gene identifier matching the historical genetic type and a value of a variant gene identifier matching the substitution gene unit as the first preset value, and set values of other identifiers in the initial type vector each as a second preset value, to generate a type vector of the substitution gene unit.

In an embodiment, the feature encoding unit is further configured to construct an initial location vector of the respective gene unit, the initial location vector comprising a plurality of candidate chromosome identifiers; and determine, from the plurality of candidate chromosome identifiers, a target chromosome identifier corresponding to the chromosome location of the respective gene unit, and set a value of the target chromosome identifier as a first preset value, and set values of other candidate chromosome identifiers in the initial location vector each as a second preset value, to generate a location vector of the respective gene unit in the chromosome location matrix.

In an embodiment, the encoding network includes a text encoding layer and a value encoding layer. The feature encoding unit is further configured to extract, based on the text encoding layer, a text feature in the multi-modal state information, and extract, based on the value encoding layer, a value feature in the multi-modal state information; and determine, according to the text feature and the value feature, the multi-modal state feature.

In an embodiment, the decoding network includes a pooling layer and a classification layer. The feature decoding unit is configured to perform, based on the pooling layer, dimensionality reduction on the multi-modal biological feature to obtain a one-dimensional abnormal sequence feature of the object; and predict, based on the classification layer, a matching probability between the one-dimensional abnormal sequence feature and each piece of candidate state information in a candidate state information set, and determine candidate state information having a matching probability falling within a preset probability interval as the disease state information.

In an embodiment, the disease state prediction apparatus includes:
a data set obtaining module, configured to obtain a state information set, the state information set comprising historical abnormal sequence information, historical multi-modal state information, and historical state information labels of a plurality of sample objects; and
a model training module, configured to predict sample disease state information according to the historical abnormal sequence information and the historical multi-modal state information by using the state prediction model, and train, according to loss values between the sample disease state information and the historical state information labels, the state prediction model.

In an embodiment, the state prediction model is a genetic disease prediction model; and, the abnormal sequence information comprises: a genetic reference sequence, a genetic variation sequence, a chromosome location sequence, and an absolute genetic location sequence of the object; the multi-modal state information comprises: clinical information and image information of the object; and the disease state information comprises: a genetic disease type of the object.

According to the foregoing disease state prediction apparatus, by performing feature extraction, analysis, and prediction on the original gene sequence and the multi-modal state information of the object by using the preset state prediction model, interference from subjective factors can be avoided to some extent, and objectivity of the disease state information is ensured. In addition, during the state prediction, the multi-modal state information of the object and the multi-modal information about the locations and the types of the abnormal gene units in the gene sequence are comprehensively considered to comprehensively and precisely represent the actual abnormal state feature of the object, so that the multi-modal biological feature extracted by the state prediction model is closer to the actual abnormal state of the object, thereby improving authenticity and accuracy of the disease state information obtained by analyzing the multi-modal biological feature by the state prediction model.

The modules in the foregoing disease state prediction apparatus may be all or partially implemented by software, hardware, or a combination thereof. The foregoing modules may be embedded in or independent of a processor in a computer device in a form of hardware, or may be stored in a memory in the computer device in a form of software, so that the processor invokes the modules and performs operations corresponding to the foregoing modules.

In an embodiment, a computer device is provided. The computer device may be a server, and an internal structural diagram of the computer device may be shown in FIG. 12. The computer device includes a processor, a memory, an input/output (I/O) interface, and a communication interface. The processor, the memory, and the input/output interface are connected through a system bus, and the communication interface is connected to the system bus through the input/output interface. The processor of the computer device is configured to provide computing and control capabilities. The memory of the computer device includes a non-volatile storage medium and an internal memory. The non-volatile storage medium stores an operating system, a computer program, and a database. The internal memory provides an environment for running of the operating system and the computer program in the non-volatile storage medium. The database of the computer device is configured to store state prediction data. The input/output interface of the computer device is configured to exchange information between the processor and an external device. The communication interface of the computer device is configured to connect to and communicate with an external terminal through a network. The computer program, when executed by the processor, implements a disease state prediction method.

In an embodiment, a computer device is provided. The computer device may be a terminal, and an internal structural diagram of the computer device may be shown in FIG. 13. The computer device includes a processor, a memory, an input/output interface, a communication interface, a display unit, and an input apparatus. The processor, the memory, and the input/output interface are connected through a system bus, and the communication interface, the display unit, and the input apparatus are connected to the system bus through the input/output interface. The processor of the computer device is configured to provide computing and control capabilities. The memory of the computer device includes a non-volatile storage medium and an internal memory. The non-volatile storage medium stores an operating system and a computer program. The internal memory provides an environment for running of the operating system and the computer program in the non-volatile storage medium. The input/output interface of the computer device is configured to exchange information between the processor and an external device. The communication interface of the computer device is configured to perform wired or wireless communication with the external terminal. The wireless communication may be implemented through WIFI, a mobile cellular network, near field communication (NFC), or another technology. The computer program, when executed by the processor, implements a disease state prediction method. The display unit of the computer device is configured to form a visually visible image, and may be a display screen, a projection apparatus, or a virtual reality imaging apparatus. The display screen may be a liquid crystal display screen or an e-ink display screen. The input apparatus of the computer device may be a touch layer covering the display screen, or may be a button, a trackball, or a touchpad disposed on a housing of the computer device, or may be an external keyboard, a touchpad, a mouse, or the like.

A person skilled in the art may understand that, the structures shown in FIG. 12 and FIG. 13 are only block diagrams of a partial structure related to the solution of the present disclosure and do not constitute a limitation to the computer device to which the solution of the present disclosure is applied. Specifically, the computer device may include more or fewer components than those shown in the figure, or some components may be combined, or a different component deployment may be used.

In an embodiment, a computer device is provided, including a memory and a processor, the memory having a computer program stored therein, and the processor, when executing the computer program, implementing operations in the foregoing method embodiments.

In an embodiment, a computer readable storage medium is provided, the computer readable storage medium having a computer program stored therein, and the computer program, when executed by a processor, implementing operations in the foregoing method embodiments.

In an embodiment, a computer program product is provided, including a computer program, the computer program, when executed by a processor, implementing operations in the foregoing method embodiments.

In conclusion, the present disclosure provides the disease state prediction method and apparatus, the device, the computer readable storage medium, and the computer program product. The computer device obtains the original gene sequence of the object, compares the original gene sequence with the reference gene sequence to obtain the abnormal sequence information that can represent the locations and the types of the abnormal gene units, obtains the multi-modal state information of the object, and inputs the abnormal sequence information and the multi-modal state information into the state prediction model for feature extraction, to finally obtain the disease state information. From a technical perspective, this mode of combining the microscopic genetic features and the macroscopic multi-modal state information of the object greatly enriches the dimensionality of input information of the model. The original gene sequence includes the most essential genetic features of the object, and the multi-modal state information reflects the actual state of the object at a microscopic level. By way of comprehensive analysis on the information at two different levels, the model can uncover the potential rules of the state of the object more comprehensively and deeply. During data processing, this mode avoids potential biases and limitations inherent in relying on a single data source, so that the model can precisely evaluate the state of the object from a plurality of perspectives, thereby significantly improving accuracy and reliability of the disease state information. In addition, the original gene sequence is preprocessed, so that the original gene sequence satisfies the identification requirement of the model, thereby ensuring that the model can run normally and efficiently, improving utilization of computing resources of the computer device, and reducing unnecessary computing overheads.

Further, when obtaining the original gene sequence of the object, first, the initial gene sequence including the gene units at the plurality of loci is obtained, and then quality filtering is performed to remove low-quality genetic data and duplicated data. The low-quality genetic data is usually introduced due to factors such as hardware errors of test devices, unstable test environments, or unregulated test methods in the sequencing process. When the erroneous data enters the subsequent analysis process, the erroneous data interferes with correct extraction of features from the gene sequence by the model, reducing accuracy of the model. Removing duplicated data can effectively reduce data redundancy and data complexity. In terms of data storage, occupation of storage space is reduced. In terms of model computation, the data processing load of the model is reduced, and the computing efficiency of the model is improved, so that the model can converge to an optimal solution more quickly, thereby improving the overall analysis speed and efficiency.

Further, when comparing the original gene sequence with the reference gene sequence to obtain the abnormal sequence information, first, sequence alignment is performed to obtain the candidate gene sequence, then the normal and abnormal gene units are determined to construct the type and location sequences, and finally, sequence alignment is performed again. The sequence alignment is a key operation in genetic analysis, and provides a basis for subsequent detailed analysis of gene units. By comparing the gene sequence with the reference gene sequence, possible abnormal regions can be quickly positioned to obtain the candidate gene sequence, thereby narrowing the analysis range and reducing unnecessary computing load. After the normal and abnormal gene units are determined, the type and location sequences are constructed. This refined information construction mode can accurately record the variation of the gene units and provide abundant and detailed information for subsequent model analysis. The sequence alignment is performed again to further verify and perfect the abnormal sequence information, thereby ensuring the accuracy and reliability of the information, and further improving the analysis precision of the model for gene abnormality.

Further, when the abnormal gene units include at least one of the newly added, deleted, and substitution gene units, the type sequence includes the genetic reference sequence and the genetic variation sequence, and the location sequence includes the chromosome location sequence and the absolute genetic location sequence, these sequences are constructed in a particular sorting and combination mode. Specifically, by identifying the insertion identifiers in the genetic reference sequence and the deletion identifiers in the genetic variation sequence, addition and deletion of gene units can be accurately determined. By comparing the genetic reference sequence with the genetic variation sequence, base types of the gene units before and after variation can be determined. By using the chromosome location sequence and the absolute genetic location sequence, the locations of the gene units in the reference gene sequence can be precisely determined. This detailed and accurate information construction mode enables the model to perform deep analysis on genetic variation from multiple dimensions, thereby providing a more accurate basis for state prediction. In the field of genetic research, accurately determining the type and location of genetic variation is of great significance to understand the occurrence mechanism of a disease, develop a personalized treatment plan, and the like.

Further, when obtaining the multi-modal state information of the object, the text, value, and image information are obtained, data mapping is performed on the text information, vectorization processing is performed on the image information, and finally, the multi-modal state information is obtained through summarization. Different forms of state information have different data formats and features in the original state, which makes processing of the model very difficult. Data mapping is performed on the text information to convert the text information into the value form, and vectorization processing is performed on the image information to unify different forms of information into value representations, so that the data formats are consistent, thereby facilitating identification and processing by the state prediction model. This unified data format not only improves processing efficiency of the model, but also avoids information loss or inaccuracy caused by different data forms, so that the model can obtain the state information of the object more comprehensively and accurately, thereby improving comprehensiveness and accuracy of the multi-modal state information.

Further, when the state prediction model includes the encoding network, the conversion network, and the decoding network, the encoding network concurrently extracts the abnormal sequence and multi-modal state features, the conversion network concatenates and performs dimensionality expansion on the features to obtain the multi-modal biological feature, and the decoding network performs dimensionality reduction to output the disease state information. The concurrent processing mode of the encoding network fully uses the multi-core computing capability of the computer device, and can perform feature extraction on the abnormal sequence and multi-modal state information at the same time, thereby greatly improving feature extraction efficiency. When processing large-scale data, concurrent computation can significantly shorten the processing time and improve the response speed of the system. By concatenating and performing dimensionality expansion on the features from different sources, the conversion network can uncover potential relationships between features in different modalities, so that the multi-modal biological feature is more representative and effective. Through the dimensionality expansion operation, the low-dimensional feature is mapped to a high-dimensional space, thereby enhancing the feature expression capability, and enabling the model to better capture complex patterns in data. The dimensionality reduction of the decoding network converts the high-dimensional multi-modal biological feature into the low-dimensional disease state information, thereby removing redundant information, reserving key features, and improving accuracy and interpretability of the prediction result.

Further, when the sequence abnormality encoding layer in the encoding network processes the abnormal sequence information, the types of the gene unit are determined by identifying the genetic reference and variation sequences, the type vectors are generated and combined into the type matrix, the chromosome location sequence is identified, the location vectors are generated and combined into the chromosome location matrix, and finally, the type matrix, the chromosome location matrix, and the absolute genetic location sequence are concatenated to obtain the abnormal feature matrix as the abnormal sequence feature. In this processing mode, the abnormal information of the gene units is structurally represented in the form of a matrix, so that the variation of different gene units can be stored and processed in a unified and standard mode. The construction of the type matrix and the location matrix makes the types and location information of the gene units clearer and more precise, thereby facilitating analysis and comparison by the model. The concatenated abnormal feature matrix integrates information about many aspects such as the types and locations of the gene units, provides rich and comprehensive feature representations for subsequent model analysis, and improves the analysis capability and prediction accuracy of the model for gene abnormality.

Further, when generating the type vector based on the type of the gene unit, the initial type vector including the particular identifiers is constructed, and the value of the corresponding identifier is set according to whether the gene unit is newly added, deleted, or substituted. This value encoding mode accurately represents the types of the gene units in a digital form, so that the variation information of the gene units can be stored and transmitted in a standardized mode. The type vectors of different gene units have the same length and format, but have different values for the identifiers. This enables the model to conveniently compare and analyze the types of different gene units. During data processing, the digital representation mode is convenient for the computer device to perform rapid computation and processing, thereby improving data processing efficiency and accuracy.

Further, when generating the location vector based on the chromosome location of the gene unit, the initial location vector including the plurality of candidate chromosome identifiers is constructed, the target chromosome identifier is determined, and the value of the target chromosome identifier is set. In this mode, the chromosome locations of the gene units are uniformly represented in a digital form, so that the chromosome location information is clearer and more accurate. The location vectors of different gene units have a uniform format, which facilitates batch processing and analysis of the model. By setting the value of the target chromosome identifier, the location of the gene unit in the chromosome can be precisely positioned, which provides accurate location information for subsequent genetic analysis and research, and improves efficiency and accuracy of processing the location information of the gene units.

Further, when the encoding network includes the text encoding layer and the value encoding layer, the text and value features in the multi-modal state information are respectively extracted, and then the extracted features are included in the multi-modal state feature. Different encoding layers specially process different types of information, so that key features in the information can be extracted more effectively. The text encoding layer can perform semantic analysis on the text information to extract the important semantic feature therein. The value encoding layer can perform statistical analysis on the value information to extract the value feature therein. The mode of concurrently extracting features fully uses the multi-core computing capability of the computer device, and improves the feature extraction speed. The extracted text and value features are included in the multi-modal state feature, so that the model can take different types of information into consideration at the same time, thereby improving comprehensive utilization efficiency of the multi-modal state information and the prediction capability of the model.

Further, when the decoding network includes the pooling layer and the classification layer, the pooling layer performs dimensionality reduction on the multi-modal biological feature to obtain the one-dimensional abnormal sequence feature, and the classification layer further performs dimensionality reduction, predicts the matching probability between the object and the candidate state information, and uses candidate state information having a matching probability falling within the preset probability interval as the disease state information. By locally aggregating the multi-modal biological feature, the dimensionality reduction of the pooling layer removes redundant information, and reserves key features, thereby reducing dimensionality and complexity of data. In terms of data storage, the demand for storage space is lowered. In terms of model computation, the computing load is reduced, and the computing efficiency is improved. The classification layer further performs dimensionality reduction and predicts the matching probability, so that the matching degree between the object and different candidate state information can be determined more precisely. By setting the preset probability interval, the most possible disease state information can be obtained, thereby improving accuracy and reliability of the prediction result.

Further, the method for training the state prediction model includes: obtaining the state information set including the historical information of the plurality of sample objects, obtaining the sample disease state information through prediction by the model, and then performing training according to the sample disease state information and the loss values of the historical state information labels. The diversified data set includes information of different objects in different cases, so that the model can learn a wider range of features and rules. The parameters of the model are constantly adjusted, so that the prediction result of the model gradually approaches a real situation, thereby improving accuracy and generalization capability of the model. The end-to-end training mode omits manual intervention, thereby reducing difficulty in obtaining the data set, and improving efficiency and automation of model training.

Further, when the state prediction model is the genetic disease prediction model, the genetic reference, variation, chromosome location, and absolute genetic location sequences of the object, and clinical and image information are obtained, and are input into the model to extract the multi-modal biological feature, and the multi-modal biological feature is analyzed to predict the genetic disease type. From a genetic perspective, an abnormality of the gene sequence may be a root cause of the occurrence of a disease, and a potential disease risk can be disclosed by analyzing gene-related sequences. The clinical information reflects the current physical condition and symptom of the patient, and the image information visually presents the internal structure and pathological condition of the body. The multi-modal information is comprehensively input into the model, so that the model can evaluate the genetic disease from multiple perspectives and more accurately capture the features and rules of the disease, thereby improving the accuracy of genetic disease prediction.

Further, in a genetic disease prediction scenario, the original gene sequence of the object is obtained, sequence alignment and variation testing are performed to obtain the abnormal sequence information, the clinical omics information and the radiomics information are obtained and input into the genetic disease prediction model, the probability of the genetic disease type is outputted. The abnormal sequence information obtained after processing the original gene sequence provides detailed abnormal conditions at a genetic level, the clinical omics information reflects the macroscopic physical state of the patient, and the radiomics information provides visual image features. By comprehensively analyzing the multi-modal information, the model can understand the condition of the patient more comprehensively, and learn relationships between various complex factors and the genetic disease, thereby making more accurate genetic disease prediction. During diagnosis and treatment of the disease, accurate genetic disease prediction can provide an important basis for a doctor to develop a personalized treatment plan, thereby improving the treatment effect and success rate.

Further, in another state prediction process, the text, value, and image information of the object are obtained and processed to obtain the multi-modal state information; the original gene sequence is obtained, and related sequences are constructed; the features are extracted by using different encoding layers; the conversion network concatenates and performs dimensionality expansion on the features; and the decoding network performs dimensionality reduction to obtain the disease state information. The network layers of the preset state prediction model perform feature extraction, analysis, and prediction on the original gene sequence and the multi-modal state information, thereby avoiding interference from subjective factors and ensuring objectivity of the prediction result. The table features of the object at a macroscopic level and the genetic fine-grained features of the object at a microscopic level are comprehensively considered to comprehensively and precisely represent the actual abnormal state of the object. From the perspective of data processing, this mode of comprehensively using the multi-modal information can fully uncover potential information in data, thereby improving the analysis capability and prediction accuracy for the state of the object.

Further, in the pangenetic disease identification scenario, the clinical omics information, the radiomics information, and the original gene sequence of the object are obtained, processed, and input into the genetic type prediction model to obtain the prediction result. By using the multi-modal biological feature, information about the object can be obtained from different omics and levels, so that the model can understand the state of the object more comprehensively. The clinical omics information provides basic physiological information and medical history of the patient, the radiomics information presents the internal structure and pathological condition of the body, and the original gene sequence discloses the abnormal condition at a genetic level. By way of comprehensive analysis on the information, the model can precisely position potential genetic disease information, thereby providing a more accurate basis for subsequent disease analysis and research. In terms of disease prevention and control, accurate genetic disease identification can help a doctor find a potential disease risk in time, and take corresponding preventive measures to reduce the incidence of the disease.

Further, in the pulmonary genetic disease identification scenario, the related information of the object is obtained, processed, and input into the pulmonary genetic disease type prediction model. The encoding layers of the model are configured to encode different types of information. The core network concatenates the features and extracts the multi-modal biological feature. The decoding network performs dimensionality reduction and matching to determine the predicted pulmonary genetic disease. The pulmonary genetic disease is analyzed from multiple dimensions, and the clinical information, the genetic information, and the image information are considered comprehensively, so that the model can more accurately capture the features and rules of the pulmonary genetic disease. In terms of disease diagnosis, diagnosis accuracy and specificity are improved. In terms of development of treatment plans, the doctor is provided with a more scientific basis, which helps develop a more effective treatment plan, and improves the treatment effect and quality of life of the patient.

User information (including, but not limited to, user equipment information, user personal information, and the like) and data (including, but not limited to, data for analysis, stored data, displayed data, and the like) involved in the present disclosure are both information and data that are authorized by a user or that are sufficiently authorized by all parties, and acquisition, use, and processing of related data need to comply with related laws and regulations and standards of related countries and regions.

Those of ordinary skill in the art may understand that all or some of the procedures in the method of the foregoing embodiments may be implemented by a computer program instructing related hardware. The computer program may be stored in a non-volatile computer-readable storage medium. When the computer program is executed, the procedures in the embodiments of the foregoing method may be performed. Any reference to a memory, a database, or other media used in the embodiments of the present disclosure may include at least one of a non-volatile and a volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, and the like. The volatile memory may include a random access memory (RAM), an external cache, or the like. By way of illustration and not limitation, the RAM may be in various forms, such as a static random access memory (SRAM) or a dynamic random access memory (DRAM). The database involved in the embodiments provided in the present disclosure may include at least one of a relational database and a non-relational database. The non-relational database may include a blockchain-based distributed database, but is not limited thereto. The processor in the embodiments of the present disclosure may be a general processor, a central processor, a graphics processor, a digital signal processor (DSP), a programmable logic device, and a data processing logic device based on quantum computing, but is not limited thereto.

Technical features of the foregoing embodiments may be randomly combined in different modes to form other embodiments. For brevity of description, not all possible combinations of the technical features in the foregoing embodiments are described.

The foregoing embodiments only describe several implementations of the present disclosure, which are described specifically and in detail, and therefore cannot be construed as a limitation to the present disclosure. A person of ordinary skill in the art may further make various changes and improvements without departing from the ideas of the present disclosure, and these changes and improvements all fall within the present disclosure.

## Claims

1. A disease state prediction method, executable by a computer device, the method comprising:
obtaining an original gene sequence of an object, and comparing the original gene sequence with a reference gene sequence to obtain abnormal sequence information, the abnormal sequence information comprising locations and types of abnormal gene units in the original gene sequence;
obtaining multi-modal state information of the object; and
extracting, by a state prediction model, an abnormal sequence feature from the abnormal sequence information and a multi-modal state feature from the multi-modal state information, and predicting, by the state prediction model, based on the abnormal sequence feature and the multi-modal state feature, disease state information of the object.

2. The method according to claim 1, wherein the obtaining an original gene sequence of an object comprises:
obtaining an initial gene sequence of the object, the initial gene sequence comprising a plurality of gene units at a plurality of loci; and
performing quality filtering on the plurality of gene units to obtain the original gene sequence comprising at least one qualified gene unit.

3. The method according to claim 1 or 2, wherein the comparing the original gene sequence with a reference gene sequence to obtain abnormal sequence information comprises:
aligning the original gene sequence and the reference gene sequence to obtain a candidate gene sequence comprising a plurality of candidate gene units at a part of loci in the original gene sequence;
comparing each of the plurality of candidate gene units with a gene unit at a same location in the reference gene sequence, to determine normal gene units and abnormal gene units in the candidate gene sequence;
constructing an type sequence and an location sequence of the object according to the reference gene sequence, the normal gene units, and the abnormal gene units; and
aligning, according to absolute locations of gene units in the reference gene sequence, the type sequence and the location sequence to obtain the abnormal sequence information.

4. The method according to claim 3, wherein the abnormal gene units comprise insertion gene units, deletion gene units, and substitution gene units; the type sequence comprises a genetic reference sequence and a genetic variation sequence; the location sequence comprises a chromosome location sequence and an absolute genetic location sequence; and the constructing an type sequence and an location sequence of the object according to the reference gene sequence, the normal gene units, and the abnormal gene units comprises:
performing, according to the absolute locations of the gene units in the reference gene sequence, the following operations:
sorting type identifiers of gene units in the reference gene sequence and insertion identifiers of the insertion gene units, to obtain the genetic reference sequence;
sorting type identifiers of the normal gene units, type identifiers of the substitution gene units, type identifiers of the insertion gene units, and deletion identifiers of the deletion gene units, to obtain the genetic variation sequence;
sorting chromosome locations of gene units in the genetic variation sequence and chromosome locations of the deletion gene units, to obtain the chromosome location sequence; and
sorting absolute genetic locations of gene units in the genetic variation sequence and absolute genetic locations of deletion gene units, to obtain the absolute genetic location sequence.

5. The method according to any one of claims 1 to 4, wherein the obtaining multi-modal state information of the object comprises:
obtaining text information, value information, and image information of the object;
mapping, according to a preset conversion relationship, the text information to a text feature of the object;
extracting an image feature from the image information; and
obtaining, based on the text feature, the image feature, and the value information, the multi-modal state information.

6. The method according to any one of claims 1 to 5, wherein the state prediction model comprises an encoding network, a conversion network, and a decoding network; and the extracting, by a state prediction model, an abnormal sequence feature from the abnormal sequence information and a multi-modal state feature from the multi-modal state information, and predicting, by the state prediction model, based on the abnormal sequence feature and the multi-modal state feature, disease state information of the object comprises:
extracting, based on the encoding network, the abnormal sequence feature and the multi-modal state feature;
concatenating, based on the conversion network, the abnormal sequence feature and the multi-modal state feature, and performing dimensionality expansion on the concatenated feature to obtain a multi-modal biological feature; and
performing, based on the decoding network, dimensionality reduction on the multi-modal biological feature to output the disease state information.

7. The method according to claim 6, wherein the extracting, by a state prediction model, an abnormal sequence feature from the abnormal sequence information comprises:
identifying the genetic reference sequence and the genetic variation sequence to determine an type of each gene unit in the abnormal sequence information, and generating, based on the type of each gene unit, a type matrix of the abnormal sequence information;
identifying the chromosome location sequence to determine a chromosome location of each gene unit in the abnormal sequence information, and generating, based on the chromosome location of each gene unit, a chromosome location matrix of the abnormal sequence information; and
concatenating the type matrix, the chromosome location matrix, and the absolute genetic location sequence to obtain the abnormal sequence feature.

8. The method according to claim 7, wherein the type matrix comprises type vectors of gene units in the abnormal sequence information, and the generating, based on the type of each gene unit, a type matrix of the abnormal sequence information comprises:
constructing an initial type vector of the respective gene unit, the initial type vector comprising an insertion gene identifier, a deletion gene identifier, a plurality of reference gene identifiers of different types, and a plurality of variant gene identifiers of different types;
setting, when the respective gene unit is an insertion gene unit, a value of the insertion gene identifier and a value of a variant gene identifier matching the type of the insertion gene unit in the initial type vector as a first preset value, and setting values of other identifiers in the initial type vector each as a second preset value, to generate an type vector of the insertion gene unit;
setting, when the respective gene unit is a deletion gene unit, a value of the deletion gene identifier and a value of a reference gene identifier matching the type of the deletion gene unit in the initial type vector as the first preset value, and setting values of other identifiers in the initial type vector each as a second preset value, to generate an type vector of the deletion gene unit; and
determining, when the respective gene unit is a substitution gene unit, a historical genetic type of the substitution gene unit, setting a value of a reference gene identifier matching the historical genetic type and a value of a variant gene identifier matching the substitution gene unit as the first preset value, and setting values of other identifiers in the initial type vector each as a second preset value, to generate an type vector of the substitution gene unit.

9. The method according to claim 7 or 8, wherein the generating, based on the chromosome location of each gene unit, a chromosome location matrix of the abnormal sequence information comprises:
constructing an initial location vector of the respective gene unit, the initial location vector comprising a plurality of candidate chromosome identifiers; and
determining, from the plurality of candidate chromosome identifiers, a target chromosome identifier corresponding to the chromosome location of the respective gene unit, and setting a value of the target chromosome identifier as a first preset value, and setting values of other candidate chromosome identifiers in the initial location vector each as a second preset value, to generate a location vector of the respective gene unit in the chromosome location matrix.

10. The method according to any one of claims 6 to 9, wherein the encoding network comprises a text encoding layer and a value encoding layer; and the extracting, by a state prediction model, a multi-modal state feature from the multi-modal state information comprises:
extracting, based on the text encoding layer, a text feature in the multi-modal state information;
extracting, based on the value encoding layer, a value feature in the multi-modal state information; and
determining, according to the text feature and the value feature, the multi-modal state feature.

11. The method according to any one of claims 6 to 10, wherein the decoding network comprises a pooling layer and a classification layer; and the performing, based on the decoding network, dimensionality reduction on the multi-modal biological feature to output the disease state information comprises:
performing, based on the pooling layer, dimensionality reduction on the multi-modal biological feature to obtain a one-dimensional abnormal sequence feature of the object; and
predicting, based on the classification layer, a matching probability between the one-dimensional abnormal sequence feature and each piece of candidate state information in a candidate state information set, and determining candidate state information having a matching probability falling within a preset probability interval as the disease state information.

12. The method according to any one of claims 1 to 11, wherein training the state prediction model comprises:
obtaining a state information set, the state information set comprising historical abnormal sequence information, historical multi-modal state information, and historical state information labels of a plurality of sample objects; and
predicting sample disease state information according to the historical abnormal sequence information and the historical multi-modal state information by using the state prediction model, and training, according to loss values between the sample disease state information and the historical state information labels, the state prediction model.

13. The method according to any one of claims 1 to 12, wherein the state prediction model is a genetic disease prediction model; and, the abnormal sequence information comprises: a genetic reference sequence, a genetic variation sequence, a chromosome location sequence, and an absolute genetic location sequence of the object;
the multi-modal state information comprises: clinical information and image information of the object; and
the disease state information comprises: a genetic disease type of the object.

14. A disease state prediction apparatus, comprising:
a sequence obtaining module, configured to obtain an original gene sequence of an object, and comparing the original gene sequence with a reference gene sequence to obtain abnormal sequence information, the abnormal sequence information comprising locations and types of abnormal gene units in the original gene sequence;
a state obtaining module, configured to obtain multi-modal state information of the object; and
a state analysis module, configured to extract, by a state prediction model, an abnormal sequence feature from the abnormal sequence information and a multi-modal state feature from the multi-modal state information, and predict, by the state prediction model, based on the abnormal sequence feature and the multi-modal state feature, disease state information of the object.

15. A computer device, comprising a memory and a processor, the memory having a computer program stored therein, and the processor, when executing the computer program, implementing operations of the method according to any one of claims 1 to 13.

16. A computer readable storage medium, having a computer program stored therein, the computer program, when executed by a processor, implementing operations of the method according to any one of claims 1 to 13.

17. A computer program product, comprising a computer program, the computer program, when executed by a processor, implementing operations of the method according to any one of claims 1 to 13.
